# EUROPÄISCHE PATENTSCHRIFT

(11) **EP 3 860 538 B1**
(45) Veröffentlichungstag und Bekanntmachung des Hinweises auf die Patenterteilung: **04.11.2026**
(21) Anmeldenummer: 19783994.7
(22) Anmeldetag: 26.09.2019
(51) Int. Cl.: A61F 13/47, A61F 13/84

(54) **WEGWERFBARE ABSORBIERENDE INKONTINENZVORLAGE MIT KENNZEICHNUNGSMITTEL**
ABSORBENT DISPOSABLE INCONTINENCE PAD HAVING IDENTIFICATION MEANS
COUCHE ABSORBANTE JETABLE POUR INCONTINENCE DOTÉE D'UN MOYEN D'IDENTIFICATION

(30) Priorität: 01.10.2018 DE 102018124265
(43) Veröffentlichungstag der Anmeldung: 11.08.2021
(73) Patentinhaber: Paul Hartmann AG, 89522 Heidenheim (DE)
(72) Erfinder: DANNENMANN, Petra, 89542 Herbrechtingen (DE); LIMAM, Deniz, 892321 Neu-Ulm (DE); BUCH, Tamara, 89077 Ulm (DE); EILERS, Jörg, 89179 Beimerstetten (DE); SCHMIDT, Ann-Cathrin, Weißenfels 06667 (DE); BEYRLE, Andreas, Nattheim 89564 (DE); KESSELMEIER, Rüdiger, 89233 Burlafingen (DE)
(74) Vertreter: Paul Hartmann AG Patents & Licensing
(86) Internationale Anmeldenummer: PCT/EP2019/076062
(87) Internationale Veröffentlichungsnummer: WO 2020/069973

(56) Entgegenhaltungen:
- WO-A1-2006/066029
- WO-A1-2007/135625
- WO-A1-2015/076189
- JP-A- 2002 360 620

## Beschreibung

Die vorliegende Erfindung betrifft eine wegwerfbare absorbierende Inkontinenzvorlage mit einer Längsrichtung und einer Querrichtung, einer Längsmittelachse und einer Quermittelachse, mit einer Produktlänge L1 und mit einer Produktbreite B1, mit einem entlang der Längsrichtung angeordneten hinterem Produktabschnitt, einem mittleren Produktabschnitt und einem vorderem Produktabschnitt, wobei jeder Produktabschnitt jeweils ein Drittel der Produktlänge L1 erfasst, mit einem hinteren Produktendabschnitt und einem vorderen Produktendabschnitt, welche in Längsrichtung jeweils ein Zehntel (1/10) der Produktlänge L1 erfassen, mit einer Innenseite und einer Außenseite, und mit einem Deckblatt-Material, einem Backsheet-Material und einem dazwischen angeordneten Saugkörper.

Wegwerfbare absorbierende Inkontinenzvorlagen weisen entlang der Längsrichtung zumeist unterschiedliche Zonen und Bereiche an Funktionalität auf, so dass die richtige Positionierung der Inkontinenzvorlage am Träger und dabei die Orientierung, welches der beiden Produktenden vorne bzw. hinten angeordnet sein soll, ganz wesentlich ist.

Zum Anlegen einer Inkontinenzvorlage, die sich herstellerseitig oftmals in einer gefalteten Konfiguration befindet, wird die Inkontinenzvorlage auseinandergefaltet und entlang der Längsrichtung mittig einwärts auf die Innenseite gefaltet - zu einem "Schiffchen" - und bezogen auf den Träger in dieser Schiffchen-Konfiguration von vorne nach hinten durch die Beine des Trägers geschoben. Die jeweiligen Produktenden werden dann plan auf den Träger ausgebreitet und die Inkontinenzvorlage wird am Träger fixiert, so mittels weiterer Hilfsmittel, wie beispielsweise durch Überziehen einer Unterhose, Überziehen eines Netzhöschens und gegebenenfalls zusätzlich mittels Haftungsmittel wie Klebestreifen.

Einer geschulten Pflegekraft ist es aus ihrer Fachkenntnis und auch Erfahrung bekannt, dass bei derartigen Inkontinenzvorlagen bei Aufsicht auf eine Oberfläche der Inkontinenzvorlage der größere Oberflächenbereich oftmals dazu bestimmt ist auf die Rückseite des Trägers ausgerichtet zu sein.

Dennoch können Inkontinenzvorlagen mit einer weniger prägnanten Differenzierung von unterschiedlichen Oberflächenbereichen an vorderem und hinterem Produktende eine Schwierigkeit darstellen.

Bei derartigen absorbierenden Inkontinenzvorlagen handelt es sich zudem auch oftmals um Produkte von einer durchaus großen flächenhaften Erstreckung, sowohl in Längsrichtung als auch in Querrichtung, so dass ein Entfalten und vollständiges Aufspannen des Produkts zum visuellen Erfassen eines vermeintlich größeren Oberflächenbereichs umständlich und zeitlich aufwändig ist. Auch aufgrund bei größeren absorbierenden Inkontinenzvorlagen durchaus optional eingebrachten Elastifizierungsmittel kann eine Dimensions- und damit Flächeneinschätzung aufgrund der mit den Elastifizierungsmittel einhergehenden Raffung erschwert sein.

Auch kann während des Anlegevorgangs der absorbierenden Inkontinenzvorlage durch ein mehrmaliges Verändern der Konfiguration der Inkontinenzvorlage, wie von einer möglicherweisen herstellerseitigen Faltungskonfiguration in eine "Schiffchen"-Konfiguration, dabei eventuell Bedarf eines situationsbedingten Umgreifens mit den Händen (wenn die Pflegeperson den Träger der Inkontinenzvorlage stützen muss) eine Irritation in der ursprünglich angedachten Orientierung der Inkontinenzvorlage auftreten.

Zudem kann bei absorbierenden Inkontinenzvorlagen eine spezielle Zuordnung eines größeren Oberflächenbereichs auch geschlechtsspezifisch anders vorgesehen sein, so dass dieser auf die Bauchseite des Trägers, so eben bei Inkontinenzvorlagen mit einer Verwendung vorgesehen für Männer, ausgerichtet ist.

Die Problematik zur Unterscheidung zwischen vorderem und hinterem Produktende wird jedoch noch verschärft, wenn die Anwendung und das Anlegen von solchen Produkten von im Umgang mit Inkontinenzvorlagen weniger geschulten Personen, oftmals außerhalb von stationären Institutionen, sondern im heimischen privaten Umfeld vorgenommen wird.

Im Stand der Technik ist bekannt, Markierungen für vorne und hinten des jeweiligen Produkts anzubringen. So ist es von regulärer Kleidung und auch regulärer Unterwäsche bekannt, eine Markierung einzubringen, die die Vorderseite oder die Rückseite kennzeichnet.

Vor allem findet sich eine derartige Markierung oftmals im hinteren Bereich, so beispielsweise in Form eines eingenähten Etiketts.

JP2002-360620 A zeigt eine absorbierende Inkontinenzvorlage mit farbigen Kennzeichen für vorne und hinten, die zumindest von der Innenseite des Produkts erkennbar sind und in weiteren Ausführungen in Bereichen ohne Saugkörper auch von außen sichtbar sein können.

Aus der WO2006/066029 A1 ist bekannt, bei Damenhygienevorlagen eine visuell wahrnehmbare Markierung als Funktionshinweis im vorderen Produktbereich anzubringen, zumal auch wenn bei der Vorlage mit einer um eine Quermittelachse asymmetrischen Kontur das breitere Produktende zur Bauchseite des Trägers orientiert sein soll.

Ausgehend von diesen Erkenntnissen aus dem Stand der Technik, ist es wünschenswert und es ist die Aufgabe der Erfindung, für derartige wegwerfbare absorbierende Inkontinenzvorlagen eine Markierung vorzusehen, die eine einfache und unaufwändige Differenzierung für das hintere Produktende unterstützt, unabhängig vom Konfigurationszustand der Inkontinenzvorlage.

Weiter besteht insbesondere auch die Aufgabe eine Markierung für eine Inkontinenzvorlage bereitzustellen, die eine gute Kennzeichnungskraft aufweist, gleichzeitig jedoch hinreichend dezent ist, wobei insbesondere von der Markierung zwei Informationen bereitgestellt werden können.

Die Erfindung löst diese Aufgabe durch eine wegwerfbare absorbierende Inkontinenzvorlage mit den Merkmalen des Anspruchs 1.

Dazu ist im hinteren Produktendabschnitt ein Kennzeichnungsmittel vorgesehen, das an der Längsmittelachse angeordnet ist, wobei das Kennzeichnungsmittel in einem ersten Bereich des hinteren Produktendabschnittes ein erstes Kennzeichen aufweist, das durch eine flächenhaft erstreckte farbige Zone gebildet ist und das eine erste Information bereitstellt, wobei die farbige Zone visuell unterscheidbar ist von einem das erste Kennzeichen umgebenden zweiten Bereich des hinteren Produktendabschnitts und wobei das Kennzeichnungsmittel zumindest bereichsweise sowohl auf der Innenseite als auch auf der Außenseite visuell erkennbar ist.

Mit der Ausgestaltung des Kennzeichnungsmittels mit einer visuell erkennbaren farbigen Zone, wobei diese zumindest bereichsweise auf der Innenseite und auf der Außenseite visuell erkennbar ist, erhält der Anwender sowohl bei der ersten In-die-Handnahme der Inkontinenzvorlage als auch über die verschiedenen die Konfiguration der Inkontinenzvorlage verändernden Zwischenschritte beim Anlegevorgang der Inkontinenzvorlage einen Hinweis über die Orientierung der Inkontinenzvorlage.

Durch die Anordnung des Kennzeichnungsmittels an der Längsmittelachse bietet dies dem Anwender einen Hinweis auf die Längsmittelachse der Inkontinenzvorlage und damit eine Orientierungshilfe für eine mittige Faltung der Inkontinenzvorlage in Längsrichtung im Wesentlichen um die Längsmittelachse bei der Formung eines "Schiffchen" für den Anlegevorgang.

Die Anordnung des Kennzeichnungsmittels innerhalb eines hinteren Produktendabschnitts, der lediglich ein Zehntel (1/10) der Produktlänge L1 erfasst, zeigt sich als vorteilhaft, da für den Anwender die Wahrnehmung des Kennzeichnungsmittels erleichtert ist im Vergleich zu weit in Richtung Quermittelachse positionierten Markierungen.

Mit "Innenseite" ist die dem Körper des Anwenders zugewandte Oberseite der Inkontinenzvorlage gemeint und "Außenseite" bezeichnet dabei die körperabgewandte Oberseite der Inkontinenzvorlage.

Unter "Visuell unterscheidbar" wird verstanden, dass die Unterscheidung bereits mit bloßem menschlichen Auge zu erkennen ist, gegebenenfalls kann die Unterscheidbarkeit weiter mit optischen Hilfsmitteln unterstützt werden.

"Visuell wahrnehmbar bzw. visuell erkennbar" ist gleichbedeutend mit "mit bloßem menschlichen Auge erkennbar".

Der verwendete Begriff "Farbe" bzw. "farbig" bezieht sich auf die visuelle Wahrnehmungseigenschaft von aus dem Lichtspektrum abgeleiteten und als solche vom Menschen über das Auge erkennbare Lichtleistung. Farben sind typischerweise bekannt wie u.a. Schwarz, Rot, Blau, Grün, Gelb und Mischungen davon.

Die Farbe kann dabei über den CIEL*a*b*-Farbraum gemessen werden, normiert in der DIN EN ISO 11664-4: 2012-06 Teil 4. Der CIEL*a*b*-Farbraum basiert im Wesentlichen auf der Darstellung einer Farbe innerhalb eines Koordinatensystems mit drei Achsen. Die Achse L* repräsentiert die Helligkeit zwischen 0 (schwarz) - 100 (weiß); die a*-Achse gibt den Grün- oder Rotanteil wider, mit negativen Werten für Grün und positiven Werten für Rot. Die b*-Achse gibt den Blau-oder Gelb-Anteil wider, dabei mit negativen Werten für Blau und positive Werte für Gelb.

Die farbige Zone hebt sich somit bei Aufsicht auf die Innenseite der Inkontinenzvorlage im hinteren Produktendabschnitt und bei Aufsicht auf die Außenseite der Inkontinenzvorlage im hinteren Produktendabschnitt von der visuell vorherrschenden Farbe der jeweiligen Aufsichtsfläche auf der Innenseite und Außenseite im hinteren Produktendabschnitt ab.

Die farbige Zone hebt sich insbesondere von der grundlegenden Einfärbung, also einer bereits vor Aufbringung des Kennzeichnungsmittels vorherrschenden Farbe eines im hinteren Produktendabschnitts für das Kennzeichnungsmittel vorgesehenen Trägerflachbahnmaterials ab, auf welches eine die farbige Zone bildende Komponente bzw. Material vorgesehen, insbesondere eingebracht und/oder aufgebracht ist.

Die flächenhaft erstreckte farbige Zone kann auf unterschiedliche Weise im Trägerflachbahnmaterial eingebracht sein. So ist es denkbar, dass die farbige Zone durch eine Beschichtung oder einen Aufdruck und/oder auf anderem Wege ausgebildet wird.

Als Trägerflachbahnmaterial für die farbige Zone des Kennzeichnungsmittels sind insbesondere Materiallagen der Inkontinenzvorlage vorgesehen, die eine die Außenseite und oder Innenseite bildende Lage bilden. Als Trägerflachbahnmaterial sind insbesondere das Deckblatt-Material und/oder das Backsheet-Material vorgesehen.

Das erste Kennzeichen des Kennzeichnungsmittels ist durch eine flächenhaft erstreckte farbige Zone gebildet. Unter Flächenhaft erstreckt wird dabei verstanden, dass die farbige Zone nicht auf einen einfachen farbigen Punkt oder eine einfache farbige Linie reduziert sein darf. Die flächenhaft erstreckte farbige Zone erscheint dabei als vollflächige farbige Fläche. Dabei kann sich die farbige Zone durchaus als ein erkennbarer Bereich mit einem darin homogen gleichmäßigen Muster an farbigen Bereichen zusammensetzen, wie z.B. einem Punktmuster oder Linienmuster.

Unter "Beschichtung" oder "Aufdruck" wird hierbei jeglicher auf die Oberfläche eines Trägerflachbahnmaterials aufgebrachter Zusatz verstanden, der zu einer visuellen, gegebenenfalls verbunden mit einer haptischen und/oder technischen Veränderung des Trägerflachbahnmaterials führt. Die Beschichtung/der Aufdruck kann als haptisch wahrnehmbar erhoben mit einer gewissen Beschichtungsdicke als einseitig aufliegender Auftrag ausgeführt sein, oder zusätzlich oder ausschließlich als ein das Trägerflachbahnmaterial durchdringender Auftrag vorgesehen sein.

Die im Stand der Technik bekannten Verfahren zum Erhalt einer farbigen Zone umfassen beispielsweise eine Direktbeschichtung mittels Hochdruck, Tiefdruck, Flachdruck, Rotationsdruck oder auch berührungsloses Beschichten wie Besprühen. Farbige Zonen können aber auch durch Einfärbungsprozesse wie Pigmentdruck, Spritzdruck oder Schablonendruck gestaltet werden.

Materialien und oder Komponenten, die einer solchen farbigen Zone zugeordnet sind, können beispielsweise ein Kleber/ Hotmeltauftrag, eine Druckfarbe als Paste oder eine Tinte sein.

Insbesondere bevorzugt ist die farbige Zone des Kennzeichnungsmittels eine Bedruckung, insbesondere eine farbige Bedruckung.

Das Kennzeichnungsmittel, das zumindest bereichsweise sowohl auf der Außenseite als auch auf der Innenseite visuell erkennbar ist, wird vorzugsweise nur durch eine einzige die farbige Zone einbringende Maßnahme bereitgestellt. So ist insbesondere vorgesehen, dass die farbige Zone nur einer einzigen Materiallage zugeordnet ist, also in einer einzigen Materiallage eingebracht, angebracht oder aufgebracht ist.

Eine Längsmittelachse der Inkontinenzvorlage verläuft in der Längsrichtung und halbiert die die maximale Erstreckung der Inkontinenzvorlage in der Querrichtung. Eine Quermittelachse wird bestimmt, indem die Inkontinenzvorlage in ihrer maximalen Erstreckung in Längsrichtung halbiert wird. Die Quermittelachse verläuft senkrecht zur Längsmittelachse.

Das Kennzeichnungsmittel ist zumindest "an der Längsmittelachse angeordnet". Damit ist gemeint, dass das Kennzeichnungsmittel visuell erkennbar in nächster Nähe der Längsmittelachse angeordnet ist, was einen Abstand des Kennzeichnungsmittels von der Längsmittelachse bis zu maximal 20% einer maximalen Quererstreckung des Kennzeichnungsmittels noch umfasst. Insbesondere ist das Kennzeichnungsmittel so angeordnet, dass das Kennzeichnungsmittel in seiner maximalen Quererstreckung die Längsmittelachse zumindest tangiert, vorzugsweise überfängt das Kennzeichnungsmittel die Längsmittelachse.

Vorzugsweise ist das Kennzeichnungsmittel hinsichtlich seiner maximalen Quererstreckung E1 mittig zur Längsmittelachse, insbesondere symmetrisch zur Längsmittelachse angeordnet.

Besonders bevorzugt ist eine im Wesentlichen symmetrisch um die Längsmittelachse angeordnete Positionierung des Kennzeichnungsmittels. Dabei soll im Wesentlichen symmetrisch noch eine Abweichung von der absoluten Symmetrie von maximal 10%, insbesondere maximal 8% und weiter insbesondere maximal 6% umfassen, ermittelt bezüglich einer Verschiebung einer Längsmittellinie des Kennzeichnungsmittels gegenüber der Längsmittelachse der Inkontinenzvorlage. Hierzu wird der maximale Abstand a zwischen der Längsmittellinie des Kennzeichnungsmittels und der Längsrandseite der Inkontinenzvorlage, der maximale Abstand b zwischen Längsmittelachse des Inkontinenzartikels und der Längsrandseite der Inkontinenzvorlage ermittelt und daraus wie folgt berechnet als b-a/b ≤ 10%, insbesondere ≤ 8%, insbesondere ≤ 6%. Abstände a und b werden dabei auf derselben in Querrichtung ausgerichteten Linie bestimmt.

Unter einer maximalen Quererstreckung E1 des Kennzeichnungsmittels versteht sich die Erstreckung des Kennzeichnungsmittels zwischen seinen jeweils äußersten Punkten in Querrichtung der Inkontinenzvorlage. Unter einer maximalen Längserstreckung F1 des Kennzeichnungsmittels versteht sich die Erstreckung des Kennzeichnungsmittels zwischen seinen jeweils äußersten Punkten in Längsrichtung der Inkontinenzvorlage.

An dieser Stelle sei ausgeführt, dass in der vorliegenden Anmeldung jegliche Bemessungsangaben hinsichtlich Quererstreckung, Längserstreckung oder Abstand von Abschnitten oder von an sich beliebigen Komponenten des Artikels in eben ausgebreitetem Zustand der den Artikel bildenden Flachmaterialien bestimmt werden. Wenn der Artikel beispielsweise durch fadenförmige Elastifizierungsmittel im sogenannten "Stretchbond-Verfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen, Abmessungen oder Abstände ermittelt. Dieser Zustand ergibt sich bei undehnbaren Chassismaterialien auf Vliesbasis oder Vlies-Folienverbundbasis oder Folienlage auf natürliche Weise.

Die Produktlänge L1 einer Inkontinenzvorlage ist die maximale Längserstreckung gemessen in mm in Längsrichtung der Inkontinenzvorlage zwischen einem hinteren maximal äußersten Abschnitt der Konturlinie der Inkontinenzvorlage bis zu einem vorderen maximalen äußersten Abschnitt der Konturlinie der Inkontinenzvorlage.

Die Produktbreite B1 einer Inkontinenzvorlage ist die maximale Querstreckung gemessen in mm in Querrichtung der Inkontinenzvorlage zwischen in einem maximalen Abstand angeordneten Längsabschnitten der Konturlinie der Inkontinenzvorlage.

"Wegwerfbar" bedeutet, dass die absorbierende Inkontinenzvorlage zum insbesondere einmaligen Gebrauch bestimmt ist. Das heißt eine wegwerfbare absorbierende Inkontinenzvorlage ist nicht dafür geeignet und vorgesehen nach Gebrauch gereinigt oder gewaschen und anschließend wiederverwendet zu werden

Der "Überhang" wird verstanden als ein Bereich von Flachbahnmaterialien, die sich außerhalb der Konturlinie des Saugkörpers erstrecken. Der Überhang umfasst die Erstreckung des den Saugkörper umgebenden Deckblatt-Materials und/oder Backsheet-Materials außerhalb der Konturlinie des Saugkörpers oder ist daraus gebildet. Es wird die maximale Erstreckung, also die von der Konturlinie des Absorptionskörpers am weitesten distal gelegene äußere Erstreckung des Deckblatt-Materials und/oder des Backsheet-Materials herangezogen wird.

Das Backsheet-Material und/oder das Deckblatt-Material kann vorteilhafterweise aus mehreren Komponenten bestehen, so beispielsweise kann das Deckblatt-Material vorteilhafterweise ein Verbund aus einem Topsheet-Material und in Längsrichtung beidseits angrenzenden Barrieremitteln, wie beispielsweise den sogenannten Cuffelementen, sein. Es wird also so verstanden, dass auch bei Verbunden, wie zusammengesetzten Deckblatt-Materialien und/oder Backsheet-Materialien, bei denen die einzelnen Lagen sich nicht kongruent überdecken, bei der Betrachtung des Überhangs die jeweils maximale, also am weitesten distal gelegene äußere Erstreckung des Verbundes bzw. der darin vorkommenden einzelnen Material-Lagen herangezogen wird.

Der hintere Quernahtabschnitt bzw. der vordere Quernahtabschnitt ist der Bereich innerhalb des hinteren Produktendabschnitts bzw. innerhalb des vorderen Produktendabschnitts (also in Längsrichtung jeweils 1/10 der Produktlänge L1) zwischen einer den Saugkörper begrenzenden Konturlinie und einer die Inkontinenzvorlage begrenzenden Konturlinie.

Der hintere Quernahtabschnitt bzw. vorzugsweise der vordere Quernahtabschnitt umfasst einen Überhang des Backsheet-Materials und/oder Deckblatt-Materials oder ist daraus gebildet, wobei der Überhang als außerhalb der Konturlinie des Saugkörpers erstreckende Bereiche des Backsheet-Materials und/oder Deckblatt-Materials verstanden werden.

In der Inkontinenzvorlage ist innerhalb des hinteren Produktendabschnitts ein hinterer Quernahtabschnitt und bevorzugterweise innerhalb des vorderen Produktendabschnitts ein vorderer Quernahtabschnitt vorgesehen, wobei der hintere Quernahtabschnitt und bevorzugterweise der vordere Quernahtabschnitt einen Überhang umfasst oder daraus gebildet ist, wobei der Überhang einen Bereich umfasst oder daraus gebildet ist, in dem das Deckblatt-Material und/oder das Backsheet-Material sich außerhalb einer den Saugkörper begrenzenden Konturlinie erstreckt.

Das Kennzeichnungsmittel ist innerhalb des hinteren Produktendabschnitts zumindest bereichsweise in dem hinteren Quernahtabschnitt angeordnet ist.

Insbesondere bevorzugt ist das Kennzeichnungsmittel vollumfänglich innerhalb des hinteren Quernahtabschnitts angeordnet.

Das Kennzeichnungsmittel weist erfindungsgemäß ein erstes Kennzeichen auf, das durch eine flächenhaft erstreckte Zone gebildet ist.

Das Kennzeichnungsmittel kann insbesondere eine Anordnung von zumindest zwei Sub-Kennzeichnungsmittel, weiter insbesondere eine Vielzahl an Sub-Kennzeichnungsmittel aufweisen.

Die funktionale Verbundenheit von mehreren Sub-Kennzeichnungsmittel zu einem Kennzeichnungsmittel ist dadurch gegeben, dass die von zwei Sub-Kennzeichnungsmittel benachbarten farbigen Zonen der jeweiligen ersten Kennzeichen auf einer parallel zur Quermittelachse in Querrichtung der Inkontinenzvorlage verlaufenden Linie angeordnet sind und dass bei einem ersten Sub-Kennzeichnungsmittel mit einer ersten maximalen Quererstreckung E1a und bei einem benachbarten zweiten Sub-Kennzeichnungsmittel mit einer zweiten maximalen Querstreckung E1b, ein Abstand M zwischen den zwei Sub-Kennzeichnungsmittel als die kürzeste Distanz in Querrichtung zwischen den benachbarten farbigen Zonen höchstens 25 %, insbesondere höchstens 20%, insbesondere höchstens 15%, insbesondere höchstens 10% der ersten oder zweiten maximalen Quererstreckung E1a oder E1b beträgt, dabei im Falle von unterscheidbarer ersten und zweiten Querstreckung E1a und E1b auf die kleinere maximale Quererstreckung von beiden maximalen Querstreckung E1a und E1b bezogen ist.

"Auf einer zur der Quermittelachse parallelen Linie angeordnet" versteht sich als Positionierung der farbigen Zone, bei der diese Linie die farbige Zone in ihrer flächenhaften Erstreckung zumindest tangiert, vorzugsweise durchkreuzt.

Insbesondere kann ein an der Längsmittelachse angeordnetes Kennzeichnungsmittel eine Anordnung aus zwei Sub-Kennzeichnungsmittel mit je einem ersten Kennzeichen aufweisen. Die zwei Sub-Kennzeichnungsmittel gleichen sich dabei insbesondere hinsichtlich eines Abstandes N1, N2 des einzelnen Sub-Kennzeichnungsmittels zur Längsmittelachse und/oder insbesondere hinsichtlich einer maximale Querstreckung E1a, E1b des jeweiligen Sub-Kennzeichnungsmittels und /oder insbesondere hinsichtlich einer vom jeweiligen Sub-Kennzeichnungsmittel eingeschriebenen Fläche und/oder insbesondere hinsichtlich einer vom ersten Kennzeichens eingeschriebenen Fläche und Kontur, also Form der farbigen Zone. Insbesondere sind die zwei Sub-Kennzeichnungsmittel im Wesentlichen zueinander symmetrisch an der Längsmittelachse angeordnet.

Der jeweilige Abstand N1, N2 des einzelnen Sub-Kennzeichnungsmittels zur Längsmittelachse beträgt höchstens 20%, insbesondere höchstens 15%, insbesondere höchstens 10% der maximalen Querstreckung des Sub-Kennzeichnungsmittels

Insbesondere bevorzugt ist vorgesehen, dass das Kennzeichnungsmittel ein Einzelelement ist. Das heißt, dass das Kennzeichnungsmittel nur ein einziges erstes Kennzeichen gebildet aus einer flächenhaft erstreckten farbigen Zone aufweist.

Das Kennzeichnungsmittel betrifft bezüglich des ersten Kennzeichens insbesondere zusammenhängende Flächen, und weist als Randumfangslinie insbesondere geschlossene Linien, die somit Flächen ausbilden, bevorzugt, wie beispielsweise Kreise, Ellipsen, Ovale, oder aber auch polygone Flächen, also Vielecke auf. D.h., es handelt sich bevorzugt um eine geometrische Figur, die durch einen geschlossenen Streckenzug umschlossen ist. Geschlossene Streckenzüge unterstützen vorteilhaft die visuelle Wahrnehmbarkeit des Kennzeichnungsmittels.

Das Kennzeichnungsmittel und damit das erste Kennzeichen kann dabei in der Ausbildung seiner Randumfangslinie eine einfache geometrische Form besitzen, insbesondere in Form einer Ellipse, eines Ovals oder eines Kreises oder eines Rechteckes oder eines Polygons ausgebildet sein.

Insbesondere bevorzugt kann die Inkontinenzvorlage mit unterscheidbaren, unterschiedlichen Formen hinsichtlich des Kennzeichnungsmittels bzw. des ersten Kennzeichens versehen sein. Aus unterschiedlichen Formen kann eine Differenzierung der Inkontinenzvorlage als Unisex-, Männer und/oder Frauen-Inkontinenzvorlage erhalten werden. So kann vorzugsweise ein Rechteck für ein Männerprodukt und eine Ellipse oder ein Oval für ein Frauenprodukt eingesetzt werden.

Besonders bevorzugt ist eine Ausführung des Kennzeichnungsmittels, wobei das Kennzeichnungsmittel ein zweites Kennzeichen umfasst, das eine zweite Information bereitstellt, und wobei das zweite Kennzeichen dadurch gebildet ist, dass in dem ersten Kennzeichen Aussparungen der farbigen Zone vorgesehen sind.

Auf diese Weise kann ein zweites Kennzeichen vorgesehen werden, ohne eine weitere farbige Zone einbringen zu müssen, die die Gefahr birgt, dass die verschiedenen Zonen nicht sauber getrennt werden können. Vielmehr wird das zweite Kennzeichen durch die visuell wahrnehmbare Beschaffenheit des innerhalb des vom Kennzeichnungsmittel überfangenden ersten Bereichs gebildet, wobei das zweite Kennzeichen sich visuell wahrnehmbar von der farbigen Zone abhebt. Hierdurch können vergleichsweise einfach und kostengünstig zwei Informationen durch ein Kennzeichnungsmittel bereitgestellt werden, die keinen zusätzlichen Arbeitsschritt, beispielsweise Einnähen eines Etiketts, benötigen, und dadurch, dass das zweite Kennzeichen durch Aussparungen gebildet wird, die innerhalb der farbigen Zone des ersten Kennzeichens angeordnet sind, können damit in gut erkennbarer und doch dezenter Weise verschiedene Informationen bereitgestellt werden.

Insbesondere durch das Einschließen innerhalb einer farbigen Zone wird die zweite Information des zweiten Kennzeichens besser lesbar, als wenn etwa eine herkömmliche Bedruckung ohne den umschließenden Bereich des ersten Kennzeichens vorgesehen wäre. Durch den sich vom zweiten Bereich des Materials visuell abhebenden ersten Bereich des ersten Kennzeichens wird ein Kontrast geschaffen, der es ermöglicht, eine vergleichsweise klein dimensionierte Ausbildung des zweiten Kennzeichens bezüglich der Wahrnehmbarkeit zu verbessern.

Insbesondere können die Aussparungen inselförmig sein. Unter "inselförmige" Aussparungen sollen dabei insbesondere solche Aussparungen verstanden werden, die von einem hinreichend breiten Rahmen der farbigen Zone umgeben sind, da dies die Erkennbarkeit der zweiten Information verbessert. Ebenfalls ist unter "inselförmigen" Aussparungen auch eine solche zu verstehen, die durch das Zusammenziehen mehrerer für sich stehender alphanummerischer oder ansonsten für den menschlichen Verstand als separat wahrnehmbare Zeichen zu einer Aussparung gebildet wird.

Die Aussparungen, insbesondere "inselförmigen" Aussparungen, weisen insbesondere alphanumerische Zeichen auf, wie u.a. Buchstaben, Ziffern oder Sonderzeichen.

Besonders bevorzugt umfasst das zweite Kennzeichen alphanummerische Zeichen, insbesondere in einer Anordnung der alphanummerischen Zeichen mit einer auf der Innenseite erkennbaren Leserichtung. Alternativ sind die alphanummerischen Zeichen bevorzugt in einer Anordnung mit einer auf der Außenseite erkennbaren Leserichtung.

Mit der Ausgestaltung der Ausnehmungen als alphanumerische Zeichen, dessen informativen Inhalt und Kontext der Anwender durch intuitive Lesen erfasst, wird die Aufmerksamkeit eines Anwenders noch mehr auf das Kennzeichnungsmittel gelenkt und damit die Wahrnehmungskraft des Kennzeichnungsmittels unterstützt.

Insbesondere ist vorgesehen, dass die visuell wahrnehmbare Beschaffenheit des Flachbahnmaterials innerhalb der Aussparungen derjenigen visuell wahrnehmbaren Beschaffenheit des Flachbahnmaterials des zweiten Bereiches, also dem das Kennzeichnungsmittel umgebenden Bereich identisch ist.

Dabei kann insbesondere vorgesehen sein, dass das Kennzeichnungsmittel auf dem Backsheet-Material, dabei auf einer der Innenseite zugewandten Oberseite oder einer der Außenseite zugewandten Oberseite des Backsheet-Materials angebracht ist, oder insbesondere bei einem Backsheet-Material umfassend eine innere Folienlage und eine äußere Vlieslage das Kennzeichnungsmitteldazwischen und dabei insbesondere auf der der Außenseite zugewandten Oberseite der Folienlage angebracht ist oder dass das Kennzeichnungsmittel auf dem Deckblatt-Material, dabei insbesondere auf einer der Außenseite zugewandten Oberseite des Deckblatt-Materials angebracht ist.

Hierdurch wird erreicht, dass kein Abrieb der farbigen Zone erfolgt, als auch Hautfreundlichkeit durch Vermeidung von direktem Hautkontakt beibehalten wird.

Unterstützt wird die visuelle Erkennbarkeit des Kennzeichnungsmittels auf der Außenseite und Innenseite dadurch, dass zwischen dem Kennzeichnungsmittel und der jeweiligen Außenseite und Innenseite in dem ersten Bereich, in dem das Kennzeichnungsmittel angeordnet ist, vorzugsweise nur eine geringe Anzahl an Materiallagen vorgesehen ist, ganz besonders bevorzugt unterscheiden sich die Anzahl der Lagen zwischen dem Kennzeichnungsmittel zur Innenseite und der Anzahl der Lagen zwischen dem Kennzeichnungsmittel zur Außenseite höchstens um 2 Lagen, insbesondere höchstens um 1 Lage.

Der Wahrnehmung des Kennzeichnungsmittels innerhalb des hinteren Produktendabschnitts ist es insbesondere zuträglich, wenn bei Betrachtung der Inkontinenzvorlage auf der Innenseite oder bei Betrachtung der Inkontinenzvorlage auf der Außenseite ein Unterschied an Ausgestaltung innerhalb des hinteren und vorderen Produktendabschnitts vorliegt, indem der vordere Produktendabschnitt entweder frei ist von farbiger Markierung oder im Falle einer farbigen Markierung dann eine für den Anwender unterscheidbare weniger prägnante Ausgestaltung der farbigen Markierungen im vorderen Produktendabschnitt gegeben ist im Vergleich zur farbigen Zone des Kennzeichnungsmittels im hinteren Produktendabschnitt.

Um diese visuelle Erkennbarkeit und das visuelle wahrnehmbare Abheben des Kennzeichnungsmittels im hinteren Produktendabschnitt weiter zu verbessern, werden definiert verortete Positionen an definierten Bereichen, ausgedrückt in Form von imaginären Rechtecken, im hinteren und vorderen Produktendabschnitt auf einer jeweiligen Oberseite, d.h. der Außenseite bzw. der Innenseite verglichen:
Dabei kann bevorzugt vorgesehen sein, dass auf der Innenseite oder dass auf der Außenseite das Kennzeichnungsmittel eine Fläche A1 mit einer maximalen Querstreckung E1 aufweist, und dass das Kennzeichnungsmittel von einem ersten imaginären Rechteck gerade einbeschrieben ist, wobei das erste imaginäre Rechteck erste und zweite Längsseiten parallel zur Längsrichtung und eine erste innere Querseite und eine erste äußere Querseite parallel zur Querrichtung aufweist, wobei die erste innere Querseite einen ersten Abstand D1 von einem hinteren Produktende aufweist und wobei die erste und zweite Längsseite eine Länge Z1 aufweisen, und dass ein zweites imaginäres Rechteck auf derselben Innenseite oder Außenseite in dem vorderen Produktendabschnitt verortbar ist, wobei das zweite imaginären Rechteck eine dritte und eine vierte Längsseite parallel zu der Längsrichtung aufweist, und eine zweite innere Querseite und eine zweite äußere Querseite parallel zu der Querrichtung aufweist, und wobei die dritte und vierte Längsseite eine Länge Z1' aufweisen, die der Länge Z1 entspricht (Z1'=Z1) und wobei die zweite innere Querseite einen Abstand D2 von einem vorderen Produktende aufweist, und dass für jede der Positionen, in die das zweite imaginäre Rechteck verortet wird, gilt:
a) entweder ist innerhalb des zweiten imaginären Rechtecks keine visuell erkennbare farbige Markierung vorhanden, oder
b) im Falle einer visuell erkennbaren farbigen Markierung innerhalb des zweiten imaginären Rechtecks die farbige Markierung eine Fläche A2 mit einem Flächenanteil von höchstens 25%, insbesondere höchstens 20%, weiter insbesondere höchstens 15%, weitere insbesondere höchstens 10 % der Fläche A1 aufweist und/oder
c) im Falle einer visuell erkennbaren farbigen Markierung innerhalb des zweiten imaginären Rechtecks, die farbige Markierung eine maximale Querstreckung E2 aufweist, wobei die maximale Querstreckung E2 kleiner ist als die maximale Quererstreckung E1 des Kennzeichnungsmittels, wobei insbesondere die maximale Quererstreckung E2 höchstens 40 %, insbesondere höchstens 30% %, weiter insbesondere höchstens 20%, weitere insbesondere höchstens 10 % der maximalen Querstreckung E1 beträgt.

Dabei sind die Positionen, in die das zweite imaginäre Rechteck verortet wird, dadurch begrenzt, dass das zweite imaginäre Rechteck im Vergleich zum ersten imaginären Rechteck parallel zur der Längsmittelachse verschoben ist und für den Abstand D2 gilt:
a) D2 ≤ 1,5 x D1 (Abstand D2 weist einen Wert von maximal das eineinhalb-fache (1,5 x) des Wertes von D1 auf) und
b) dabei die zweite äußere Querseite des zweiten imaginären Rechtecks sich nicht über das vordere Produktende hinauserstreckt.

Unter dem hinteren bzw. vorderen Produktende wird der äußerste Punkt der maximalen Erstreckung der Inkontinenzvorlage in ihrer Längsrichtung, d.h. ein äußerster Punkt der Konturlinie des Produkts im hinteren Produktendabschnitt bzw. im vorderen Produktendabschnitt verstanden. Durch diesen äußersten Punkt wird eine zur Quermittelachse parallele Hilfsachse durchgelegt und dann der Abstand D1 bzw. D2 als Distanz in Längsrichtung zwischen der ersten inneren Querkante bzw. zwischen der zweiten inneren Querkante und der jeweiligen nächstgelegenen Hilfsachse bestimmt.

Mit einer Asymmetrie an visuell wahrnehmbarer farbiger Ausgestaltung zwischen einer im hinterem Produktendabschnitt durch das Kennzeichnungsmittel und dessen farbiger Zone eingenommenen Fläche und einem entsprechenden Bereich im vorderen Produktendabschnitt wird der Anwender vorteilhaft in der visuellen Wahrnehmbarkeit des Kennzeichnungsmittel im hinteren Produktendabschnitts unterstützt.

Die Unterschiede in einer maximalen Quererstreckung E1 des Kennzeichnungsmittels im hinteren Produktendabschnitt und einer maximalen Querstreckung E2 einer farbigen Markierung im vorderen Produktendabschnitt sind alternativ oder ergänzend zur Flächenbetrachtung für die visuelle Unterscheidungs- und Wahrnehmungskraft des Kennzeichnungsmittels vorteilhaft. Eine Quererstreckung des Kennzeichnungsmittels bzw. einer farbigen Markierung in Querrichtung und damit senkrecht zur Längsrichtung der Inkontinenzvorlage wird vom Anwender bei an sich zumeist aufgrund einer die Produktbreite B1 übertreffenden größeren Produktlänge L1 und dadurch als eher langgestreckt erscheinenden Inkontinenzvorlagen als auffallendes Merkmal erkannt als im Vergleich bei derart langgestreckt erscheinenden Inkontinenzvorlagen eine Längserstreckung des Kennzeichnungsmittel bzw. einer Markierung in Längsrichtung der Inkontinenzvorlage wahrgenommen wird.

Auch in Anbetracht von im Anwendungsvorgang vorgenommenen Konfigurationsänderungen der Inkontinenzvorlage, so z.B. bei der Schiffchen-Konfiguration durch Umfalten entlang der Längsrichtung, wodurch eine Langerstreckung des Produkts noch weiter betont wird, trägt ein dazu in Querrichtung ausgerichtetes Kennzeichnungsmittel vorteilhaft zu dessen Wahrnehmung bei.

Insbesondere ist vorgesehen, dass das Kennzeichnungsmittels bzw. die farbige Zone des ersten Kennzeichens des Kennzeichnungsmittels eine maximale Quererstreckung E1 von höchstens 1/2, insbesondere höchstens 1/3, insbesondere höchstens ¼ einer maximalen Quererstreckung B2 des hinteren Produktabschnitts, und insbesondere mindestens 1/6 einer maximalen Querstreckung B2 des hinteren Produktabschnitts aufweist.

Mit einer gewissen Limitierung des Kennzeichnungsmittels in seiner maximalen Quererstreckung bei einer der Längsmittelachse zugewandten Anordnung des Kennzeichnungsmittels wird für den Anwender die Wahrnehmbarkeit des Kennzeichnungsmittels als ein Indikator für den hinteren Bereich der Inkontinenzvorlage vorteilhaft unterstützt, zudem aus herkömmlicher Unterwäsche bekannten Markierung im Rückenbereich ein farbige Zone im hinteren Produktendabschnitt vom Anwender als weniger unangenehm wahrgenommen wird.

Weiter bevorzugt weist das Kennzeichnungsmittel eine maximale Längserstreckung F1 von höchstens 2/12, bevorzugt höchstens 1/10 der Produktlänge L1, und insbesondere mindestens 1/24 der Produktlänge L1 auf.

Mit einer Limitierung der maximalen Längserstreckung des Kennzeichnungsmittels wird die Prägnanz des Kennzeichnungsmittels für den hinteren Produktbereich unterstützt, als weiter in Richtung Quermittelachse reichende Markierungen für den Anwender andere Assoziationen oder Hinweise darstellen könnten, die der Anwender beispielsweise in einen funktionellen Zusammenhang mit dem mittleren Produktabschnitt bringen könnte und somit von der Wahrnehmbarkeit des Kennzeichnungsmittels und dessen Funktion als Indikator für den hinteren Produktabschnitt ablenken würden.

Bei der Ausgestaltung des Kennzeichnungsmittels ist insbesondere vorgesehen, dass eine maximale Querstreckung E1 des Kennzeichnungsmittels in Querrichtung der Inkontinenzvorlage größer ist als eine maximale Längserstreckung F1 des Kennzeichnungsmittels in Längsrichtung der Inkontinenzvorlage.

Bei der Handhabung einer an sich ohnehin länglich erscheinenden Inkontinenzvorlage wirkt ein Kennzeichnungsmittel mit einer in Querrichtung der Inkontinenzvorlage ausgerichteten vergleichsweise größeren maximalen Querstreckung als dessen Längserstreckung in Längsrichtung der Inkontinenzvorlage prägnanter und dem Kennzeichnungsmittel kommt damit eine größere Wahrnehmungskraft zu.

Bevorzugt ist das Kennzeichnungsmittel mit einer maximalen Längserstreckung F1 mit mindestens 50% der maximalen Längserstreckung F1, vorzugsweise mindestens 60%, weitere vorzugsweise mindestens 70%, weiter vorzugsweise mindestens 80%, weiter vorzugsweise mindestens 90% innerhalb eines hinteren Quernahtabschnitts angeordnet, insbesondere bevorzugt ist das Kennzeichnungsmittel (70) vollumfänglich innerhalb eines hinteren Quernahtabschnitts angeordnet.

Diese zumindest bereichsweise Positionierung des Kennzeichnungsmittels innerhalb eines hinteren Quernahtabschnitts, welcher definitionsgemäß keinen Saugkörper aufweist, unterstützt vorteilhaft die visuelle Erkennbarkeit des Kennzeichnungsmittels sowohl auf der Außenseite und der Innenseite der Inkontinenzvorlage. Eine zunehmende Anordnung des Kennzeichnungsmittels ausschließlich innerhalb des hinteren Quernahtabschnitts und die damit einhergehende Übereinstimmung der visuell wahrnehmbaren Flächenabdeckung des Kennzeichnungsmittels auf der Innenseite und der Außenseite, erleichtert für den Anwender das Erkennen des Kennzeichnungsmittels unabhängig von der in der Anwendung bedingten Konfigurationsänderungen der Inkontinenzvorlage zwischen Außenseite und Innenseite. Zudem wird durch Positionierung des Kennzeichnungsmittels innerhalb eines Bereiches der Inkontinenzvorlage mit einem gleichen Lagenaufbau ein einheitliches Erscheinungsbild des Kennzeichnungsmittels geschaffen, was dessen Wahrnehmbarkeit unterstützt.

Es ist insbesondere vorgesehen, dass das Kennzeichnungsmittel außerhalb eines Überlappungsbereiches mit dem Saugkörper vorgesehen ist. Hierdurch wird erreicht, dass das Kennzeichnungsmittel nicht in einem Bereich vorgesehen ist, der aufgrund der Materiallagenhäufung eine relative hohe Opazität aufweist.

Die Inkontinenzvorlage kann unterschiedliche Formen aufweisen, wobei die Form durch die äußere Kontur, also die Konturlinie der Inkontinenzvorlage gegeben ist.

Die Inkontinenzvorlage kann insbesondere eine über die Längsrichtung variierende Konturlinie der Inkontinenzvorlage aufweisen. Insbesondere ist die Inkontinenzvorlage in ihrer Form nicht symmetrisch bezogen auf die Quermittelachse. So kann eine maximale Quererstreckung B2 innerhalb des hinteren Produktabschnitts verschieden sein zu einer maximalen Querstreckung B3 des vorderen Produktabschnitts.

Vorzugsweise weist der hintere Produktabschnitt eine maximale Querstreckung B2 und der vordere Produktabschnitt eine maximale Querstreckung B3 auf, wobei ein Quotient von B2/B3 entweder
**a) einen Wert** ≥ 1 und vorzugsweise einen Wert ≤ 1,50, vorzugsweise ≤ 1,45, vorzugsweise ≤ 1,40, vorzugsweise ≤ 1,35, vorzugsweise ≤ 1,30, vorzugsweise ≤ 1,25 aufweisen kann, oder
b) einen Wert ≤ 1 und vorzugsweise ≥ 0,75, vorzugsweise ≥ 0,80, aufweisen kann.

Vor allem bei Inkontinenzvorlagen mit geringen Differenzen der maximalen Querstreckung zwischen hinterem und vorderem Produktabschnitt ist die Bereitstellung eines Kennzeichnungsmittels im hinteren Produktendabschnitt eine Hilfestellung für den Anwender, oder insbesondere auch in Fällen wie unter b) beschrieben, in denen der vordere Produktabschnitt breiter ist als der hintere Produktabschnitt, wie es durchaus für Männerprodukte sein kann.

Die Inkontinenzvorlage weist insbesondere im mittleren Produktabschnitt oder im vorderen Produktabschnitt eine gegenüber der Produktbreite B1 minimale Quererstreckung auf. Insbesondere sind im mittleren Produktabschnitt keine in Querrichtung vorstehenden Seitenabschnitte bzw. Flügel vorgesehen.

Die Inkontinenzvorlage kann über deren Produktlänge unterschiedliche Eigenschaften und damit auch unterschiedliche Funktionsbereiche aufweisen.

Insbesondere kann eine mittels der Quermittelachse abgegrenzte vordere und hintere Produktlängshälfte zumindest hinsichtlich einer Eigenschaft nicht spiegelsymmetrisch bezogen auf die Quermittelachse sein, dabei kann die Eigenschaft aus der Gruppe enthaltend Fläche des Produkts, Fläche des Saugkörpers, Lagenaufbau des Saugkörpers, Saugleistung des Saugkörpers, chemische Zusammensetzung des Saugkörpers entnommen sein.

Der Saugkörper ist geeignet und dazu bestimmt Körperausscheidungen, insbesondere Körperflüssigkeiten, insbesondere Urin aufzunehmen und dauerhaft zu speichern. Der Saugkörper kann hierzu vorteilhaft superabsorbierendes Polymermaterial (SAP) enthalten, insbesondere zu 5- 50 Gewichtsprozent, vorzugsweise zu -10 - 40 Gewichtsprozent bezogen auf das Gesamtgewicht des Saugkörpers. Das SAP-Material kann beispielsweise partikelförmig oder faserförmig oder blatt- oder schaumförmig ausgebildet sein. Das SAP-Material kann insbesondere zumindest das 15-fache, insbesondere das 20-fache seines Gewichts an 0,9 gewichtsprozentiger Kochsalzlösung absorbieren (gemessen nach NWSP 242.0.R2(15)). Im Saugkörper können alternativ oder ergänzend weitere Materialien, wie Zellstofffasern (wood pulp) oder Kunststofffasern enthalten sein.

Der Saugkörper kann auch mehrere Lagen aufweisen, die sich in ihrer materiellen und/oder prozentualen Zusammensetzung (SAP, Fasertyp, chemische Faserzusammensetzung) unterscheiden, und die insbesondere zudem eine in Quer- und auch Längsrichtung unterschiedliche Erstreckung aufweisen können.

Dem Saugkörper nicht zugerechnet werden Lagen, die nicht der dauerhaften Flüssigkeitsspeicherung dienen, sondern der Flüssigkeitsverteilung und Weiterleitung, wie dem Fachmann bekannte Flüssigkeitsverteilerauflagen.

Zur Ermittlung der Saugleistung kann die Inkontinenzvorlagen bzw. deren hintere und vordere Produktlängshälfte nach der ISO 11984-1 (1996) vermessen werden.

Bei derart unterschiedlichen Eigenschaften innerhalb der hinteren und vorderen Produktlängshälfte der Inkontinenzvorlage ist ein Kennzeichnungsmittel im hinteren Produktendabschnitt eine für den Anwender wesentliche Hilfestellung zur nutzungsgerechten und der jeweiligen Inkontinenzvorlage zugeschriebenen Verwendung und Orientierung vorne/hinten.

Die Inkontinenzvorlage ist insbesondere eine Erwachsenen-Inkontinenzvorlage. Insbesondere bei mit einer Ausführungsform als eine Erwachsenen-Inkontinenzvorlage einhergehenden größeren Abmessungen, sowohl in Längsrichtung als auch in Querrichtung und in der gesamten flächenhaften Erstreckung, vermag das Kennzeichnungsmittel durch dessen Positionierung innerhalb des hinteren Produktendabschnitt vorteilhaft zu einer Differenzierung zwischen hinterem und vorderen Ende des Produkts beitragen.

Die Inkontinenzvorlage weist insbesondere eine Produktfläche von 110.000 - 260.000 mm², vorzugsweise 125.000 - 230.000 mm², weiter vorzugsweise 125.000 - 200.000 mm² auf.

Die Produktlänge L1 der Inkontinenzvorlage beträgt insbesondere 450 - 800 mm, vorzugsweise 500 - 800 mm, weiter vorzugsweise 500 - 750 mm.

Die Produktbreite B1 der Inkontinenzvorlage beträgt insbesondere 200 - 450 mm, vorzugsweise 250 - 450 mm, weiter vorzugsweise 280-400 mm.

Ein Überhang von Deckblatt-Material und/oder Backsheet-Material, das sich außerhalb einer den Saugkörper begrenzenden Konturlinie erstreckt, kann einen Flächenanteil von insbesondere 18 - 35 %, vorzugsweise 18- 32%, weiter vorzugsweise 20 - 30 % bezogen auf die Produktfläche aufweisen. Insbesondere weist ein Überhang eine Fläche von 24.000 - 56.000 mm², vorzugsweise 28.000 - 54.000 mm², weiter vorzugsweise 30.000-52.000 mm² auf.

Vorzugsweise weist ein hinterer Quernahtabschnitt einen Flächenanteil von 3- 8%, bevorzugt 4 - 7 %, weiter bevorzugt 4- 6 % bezogen auf eine Produkt-Fläche der Inkontinenzvorlage auf.

Ein vorderer Quernahtabschnitt kann einen Flächenanteil von 3- 8%, bevorzugt 4 - 7 %, weiter bevorzugt 4- 6 % bezogen auf Produktfläche der Inkontinenzvorlage aufweisen.

Insbesondere weist ein hinterer Quernahtabschnitt einen Flächenanteil von 45 - 65%, bevorzugt 48 -63 %, weiter bevorzugt 50 - 60% bezogen auf eine Fläche des hinteren Produktendabschnitts auf.

Vorzugsweise weist ein hinterer Quernahtabschnitt eine Fläche von 4.000 - 14.000 mm², vorzugsweise 5.000 - 12.000 mm², weiter vorzugsweise 5.000 -11.000 mm² auf.

Der vordere Quernahtabschnitt kann in Flächenanteil und/oder der Fläche gemessen in mm² dem hinteren Quernahtabschnitt entsprechen.

Im Falle eines hinteren Quernahtabschnitts weist dieser somit insbesondere eine gewisse flächenhafte Erstreckung zur Positionierung eines Kennzeichnungsmittels auf. Ausgedrückt als eine Breite des Quernahtabschnitts an jeglichen Punkt von der Konturlinie des Saugkörpers, und dabei die kürzeste Distanz in der Ebene der Inkontinenzvorlage von der Saugkörper-Konturlinie zur Produktkonturlinie vermessen, so beträgt die Minimum Breite vorzugsweise mindestens 20 mm, vorzugsweise mindestens 25 mm, insbesondere mindestens 30 mm und die maximale Breite beträgt vorzugsweise höchstens 80 mm, weiter vorzugsweise höchstens 70 mm, weiter vorzugsweise höchstens 60 mm.

Vorzugsweise ist vorgesehen, dass das Kennzeichnungsmittel 15- 80%, vorzugsweise 20 - 70 %, weiter vorzugsweise 25 - 60%, weiter vorzugsweise 30 - 50% einer Fläche des hinteren Quernahtabschnitts überfängt, also überlappt.

Vorzugsweise überfängt das Kennzeichnungsmittel 8 - 60%, insbesondere 10 - 55%, weiter insbesondere 10 - 50%, weiter insbesondere 10 - 40 % einer Fläche des hinteren Produktendabschnitts. (im Sinne von überlappt)

Das Kennzeichnungsmittel weist eine vorzugsweise von einer Randumfangslinie des Kennzeichnungsmittels umschriebene Fläche A1 von vorzugsweise 800 - 8000 mm², insbesondere von 900 - 6000 mm², weiter insbesondere von 1000 - 5000 mm², weiter insbesondere 1000 - 4000 mm², weiter insbesondere 1000 - 3000 mm² auf, wobei die Fläche A1 in einem planen ausgebreiteten Zustand der Inkontinenzvorlage gemessen ist.

Vorzugweise sind in einem hinteren und/oder vorderen Quernahtabschnitt das Deckblatt-Material und Backsheet-Material adhäsiv, thermisch und/oder mittels Ultraschall miteinander verbunden.

Die Inkontinenzvorlage kann zwischen einem hinteren und vorderen Quernahtabschnitt, beidseits einen sich in Längsrichtung erstreckenden ersten und einen zweiten Längsnahtabschnitt aufweisen, also einen Bereich zwischen der in Längsrichtung verlaufenden Konturlinie des Saugkörpers und der Konturlinie der Inkontinenzvorlage.

Insbesondere bevorzugt sind in einem ersten und zweiten Längsnahtabschnitt, der in Längsrichtung je beidseits der in Längsrichtung verlaufenden Konturlinie des Saugkörpers zwischen einem vorderen und einem hinteren Quernahtabschnitt angeordnet ist, keine farbigen visuell erkennbaren Markierungen vorgesehen.

Das Backsheet-Material umfasst insbesondere eine Folie oder ist daraus gebildet. Die Folie weist insbesondere ein Flächengewicht von 7 - 40 g/m², weiter insbesondere von 9 - 30 g/m², weiter insbesondere von 9 - 25 g/m² auf.

Die Folie des Backsheet-Materials kann vorteilhaft auch mit einer Vliesbeschichtung versehen sein oder mit einer der Außenseite zugewandten Vlieslage laminiert sein, was eine textile Anmutung der körperabgewandten Außenseite der Inkontinenzvorlage vermitteln kann. Die Vliesbeschichtung bzw. Vlieslage umfasst oder besteht vorzugsweise aus einem Spinnvlies (S), Meltblownvlies (M), SM-Vlies, SMS-Vlies, SMMS-Vlies, Kardenvlies oder Through Air bonded Kardenvlies, insbesondere einem Spinnvlies aus Polypropylen. Insbesondere weisen die Vlieslage/Vliesbeschichtung des Backsheet-Materials ein Flächengewicht von 5-25 g/m², 7 - 20 g/m², insbesondere von 8 - 15 g/m² auf.

Auf einer der Außenseite zugewandten Oberseite des Backsheet-Materials sind insbesondere keine adhäsiven Haftungsmittel, wie Klebeabschnitte, Klebestreifen vorgesehen.

Das Deckblatt-Material umfasst vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese.

Das Deckblatt-Material kann dabei vorzugsweise nur aus Topsheet-Material gebildet sein.

Das Topsheet-Material umfasst vorzugsweise Vliesstoffmaterialien, wie Spinnvliese, Kardenvliese oder Through Air bonded Kardenvliese. Besonders bevorzugt umfasst das Topsheet-Material Spinnvlies. Die für das Topsheet eingesetzten Vliesstoffmaterialien haben weiter vorteilhafterweise ein Flächengewicht von 5 - 20 g/m², 8 - 20 g/m², weiter vorzugsweise von 10 - 18 g/m², insbesondere vorzugsweise von 12 - 16 g/m². Besonders bevorzugt umfasst das Topsheet ein hydrophilisiertes Spinnvlies, insbesondere aus Polypropylen, insbesondere mit einem Flächengewicht von 12 - 16 g/m².

Weiter vorzugsweise kann das Deckblatt-Material ein Verbund aus Topsheet-Material und Barrieremittel-Material sein. In einer weiteren vorteilhaften Ausbildung ist das Deckblatt-Material ein Verbund aus einem flüssigkeitsdurchlässigen Topsheet-Material mit Längsrändern und angrenzenden Längsrandbereichen und beidseits an den Längsrändern bzw. Längsrandbereichen des Topsheet-Materials in Fügestellen angefügten hydrophoben Barrieremitteln. Durch diesen Verbund werden in einer Inkontinenzvorlage die bereichsweise unterschiedlichen Anforderungsprofile, nämlich im mittigen Bereich eine Flüssigkeitsaufnahme und an den Randbereichen eine Retardierung des seitlichen Flüssigkeitsaustritts, bereitgestellt.

Das Barrieremittel-Material umfasst vorzugsweise Vliesstoffmaterialien, wie Spinnvliese, Meltblownvliese, insbesondere bevorzugt Laminate aus einer oder mehreren Spinnvlies(S) und/oder Meltblown(M)-Vlieslagen, insbesondere SMS-Laminate oder SMMS-Laminate, insbesondere auf Basis von Polyolefinen, wie beispielsweise Polyethylen oder Polypropylen.

Die für das Barrieremittel eingesetzten Vliesstoffmaterialien haben weiter vorteilhafterweise ein Flächengewicht von 5 - 20 g/m², vorzugsweise von 8 - 18 g/m² , weiter vorzugsweise von 10 - 15 g/m².

Weiter vorzugsweise kann die Inkontinenzvorlage auf der Innenseite beidseits jeweils ein eine seitliche Auslaufsperre bildende und/oder beidseits entlang einer Längserstreckung des Saugkörpers verlaufendes Cuffelement aufweisen. Das Cuffelement umfasst vorzugsweise ein Vliesmaterial, insbesondere ein hydrophobes Vliesmaterial, oder ist daraus gebildet. Das Flächengewicht des hierfür eingesetzten Vliesmaterials beträgt vorzugsweise 5 - 20 g/m², vorzugsweise von 8 - 18 g/m² , weiter vorzugsweise von 10 - 15 g/m².

Das Cuffelement -Material kann das vorgenannte Barrieremittel-Material umfassen oder daraus gebildet sein.

Weiter vorzugsweise weisen die Cuffelemente eine Cuffelastifizierung auf, um ein sicheres Emporerheben und Aufstellen der Cuffelemente zu gewährleisten. Eine Elastifizierung erfolgt dabei entweder über eine flächenhafte Elastifizierung der Cuffelemente, indem die Cuffelemente in Längsrichtung elastisch dehnbare Flächenmaterialien umfassen. Insbesondere kann vorgesehen sein, dass in die Cuffelemente im Bereich ihres freien emporerhebbaren Längsrandes im Wesentlichen fadenförmige Elastifizierungsmittel eingesetzt werden oder streifenförmige Elastifizierungsmittel.

Vorzugsweise erstrecken sich Deckblatt-Material und/oder Backsheet-Material vorzugsweise zumindest in Längsrichtung der Inkontinenzvorlage, weitere vorzugsweise in Querrichtung der Inkontinenzvorlage über Konturränder /die Konturlinie des Saugkörpers hinaus, um einen entsprechenden Überhang zu bilden. In dem Überhang sind Backsheet-Material und Deckblatt-Material vorzugsweise zumindest bereichsweise miteinander verbunden, insbesondere durch an sich bekannte Fügeverfahren wie Schweißen, Siegeln, Nähen oder Kleben.

Vorzugsweise weist die Inkontinenzvorlage, insbesondere im mittleren Produktabschnitt, in Längsrichtung verlaufende Schrittelastifizierungsmittel auf. Die Schrittelastifizierungsmittel sind insbesondere zumindest abschnittsweise außerhalb der Konturlinie des Saugkörpers angeordnet.

Die Schrittelastifizierungsmittel können elastische Fäden oder auch flachmaterial- oder bandförmige Materialien wie elastische Bänder, Folien, Vliesstoffe oder Schaumstoffe aufweisen. "In Längsrichtung der Inkontinenzvorlage" bedeutet hierbei, dass das Schrittelastifizierungsmittel zumindest eine Komponente in Längsrichtung der Inkontinenzvorlage aufweist, mithin auch schräg oder gekrümmt zur Längsrichtung der Inkontinenzvorlage verlaufen kann. Die mit den Schrittelastifizierungsmittel versehenen Bereiche der Inkontinenzvorlage können als zusätzliche seitliche Auslaufsperre dienen und gleichzeitig das Anlegen der Inkontinenzvorlage, insbesondere des mittleren Produktabschnitts gegen den Körper eines Trägers fördern und eine bootförmige Krümmung der Inkontinenzvorlage hervorrufen.

Vorzugsweise sind im hinteren und/oder vorderen Produktendabschnitt, weiter insbesondere im hinteren und/oder vorderen Quernahtabschnitt keine in die Querrichtung verlaufende Elastifizierungsmittel vorgesehen. Insbesondere sind im Bereich des Kennzeichnungsmittels keine in Querrichtung verlaufende Elastifzierungsmittel vorgesehen. Somit wird der Bereich mit dem Kennzeichnungsmittel keinen durch die Elastifizierung bedingten unterschiedlichen Dehnungszuständen unterworfen und kann somit einen einheitlichen Farbeindruck vermitteln.

Nach einer weiteren bevorzugten Ausführungsform kann vorgesehen sein, dass die Inkontinenzvorlage zum Vertrieb oder im beim Vertrieb dargebotenen Zustand herstellerseitig in eine gefaltete Konfiguration gebracht ist. Vorzugsweise ist die gefaltete Konfiguration derart, dass die Innenseite der Inkontinenzvorlage in gefalteten Konfiguration keine äußere Oberseite der gefalteten Konfiguration bildet.

Dabei kann insbesondere vorgesehen sein, dass die Inkontinenzvorlage herstellerseitig in eine gefaltete Konfiguration verbracht ist, wobei die Inkontinenzvorlage zumindest um eine in Querrichtung der Inkontinenzvorlage verlaufende Faltachse gefaltet ist, und die Innenseite der Inkontinenzvorlage auf sich gefaltet in der gefalteten Konfiguration innen angeordnet ist.

Dabei wird die Inkontinenzvorlage insbesondere um eine im Wesentlichen entlang der Quermittelachse verlaufenden Faltachse gefaltet

Weiter kann vorgesehen sein, dass die Inkontinenzvorlage herstellerseitig in eine gefaltete Konfiguration verbracht ist, wobei die Inkontinenzvorlage um zwei in Querrichtung verlaufende Faltachsen unter Erhalt eines in Längsrichtung hinteren Faltungsabschnitts, der den das Kennzeichnungsmittel aufweisenden hinteren Produktendabschnitt umfasst, eines mittleren und eines vorderen Faltungsabschnitts gefaltet ist, wobei der vordere Faltungsabschnitt in der gefalteten Konfiguration zwischen dem hinteren Faltungsabschnitt und dem mittleren Faltungsabschnitt angeordnet ist oder wobei der hintere Faltungsabschnitt in der gefalteten Konfiguration zwischen dem vorderen Faltungsabschnitt und dem mittleren Faltungsabschnitt angeordnet ist.

Ergänzend zu Faltungen entlang der Querrichtung, kann auch vorgesehen sein, dass je beidseits der Längsmittelachse eine im Wesentlichen in Längsrichtung erstreckte Längsfaltachse vorgesehen ist, um welche seitliche Bereiche der Inkontinenzvorlage, insbesondere seitlich vorstehende Bereiche des hinteren und/oder vorderen Produktabschnitts der Inkontinenzvorlage in Richtung auf die Längsmittelachse auf die Innenseite eingefaltet sind.

Insbesondere bevorzugt kann eine Inkontinenzvorlage in einer herstellerseitig verbrachten gefalteten Konfiguration vorgesehen sein, mit beidseits der Längsmittelachse seitlichen Bereichen der Inkontinenzvorlage, die um je ein Längsfaltachse auf die Innenseite eingefaltet sind und nach dieser Faltungszwischenkonfiguration, um zumindest eine in Querrichtung, insbesondere um zwei in Querrichtung verlaufende Faltachsen auf die Innenseite eingefaltet ist, wobei das Kennzeichnungsmittel im hinteren Produktendabschnitt vorgesehen ist.

Bei Einfaltung um eine Längsfaltachse überlappen die eingefalteten seitlichen Bereiche der Inkontinenzvorlage, insbesondere die seitlichen Bereiche des hinteren Produktabschnitts nicht mit dem Kennzeichnungsmittel, d.h. die eingefalteten seitlichen Bereiche enden außerhalb des vom Kennzeichnungsmittel eingeschriebenen ersten Bereichs.

Über die Gestaltung des Kennzeichnungsmittels kann neben der ersten und/oder der zweiten Information eine weitere Information dadurch transportiert werden, dass die farbige Zone neben der Information, wo der hintere Bereich der Inkontinenzvorlage sich befindet, eine weitere Information beinhalten kann, indem beispielsweise über die gezielte Auswahl einer Farbe der farbigen Zone des ersten Kennzeichens ein Hinweis auf eine Saugstärke beinhaltet sein kann.

Das erste Kennzeichen kann weitere Information beinhalten, indem beispielsweise über die gezielte Auswahl einer Farbe der farbigen Zone ein Hinweis über eine Verwendung in geschlechtsspezifischer Weise beinhaltet sein kann. So kann beispielsweise ein Kennzeichnungsmittel in einer blauen Farbe auf einen bevorzugten Einsatzbereich für Männer und ein Kennzeichnungsmittel in einer rosa Farbe auf einen bevorzugten Einsatz für Frauen hinweisen oder mit einer weiteren davon verschiedenen Farbe auf die Verwendung als Unisex-Inkontinenzvorlage hinweisen.

Weiter kann über die Form des Kennzeichnungsmittels bzw. über die Form des ersten Kennzeichens, wie beispielsweise Ellipse versus Rechteck, eine weitere Information vermittelt werden.

Als zweites Kennzeichen können dann durch die Aussparungen weitere Informationen transportiert werden. Durch die Aussparungen in der farbigen Zone des ersten Kennzeichens greift das zweite Kennzeichen, die visuell wahrnehmbare Beschaffenheit, wie eben die Farbe des Trägerflachbahnmaterials des zweiten Bereichs auf. Hierdurch wird eine unerwünschte durch visuelle Erscheinung oder Auffälligkeit erzeugte Aufmerksamkeit reduziert, da sich mit einer identischen visuell wahrnehmbaren Beschaffenheit des Trägerflachbahnmaterials in den Aussparungen in Zusammenschau mit dem Trägerflachbahnmaterial des das Kennzeichnungsmittel umgebenden zweiten Bereichs ein gewisses homogenes Erscheinungsbild ergibt.

Dabei ist besonders bevorzugt vorgesehen, dass die farbige Zone ausgehend von einer Randumfangslinie des Kennzeichnungsmittels das zweite Kennzeichen derart umschließt, dass die Größe einer Fläche A3, welche von der Randumfangslinie des Kennzeichnungsmittels und einer davon innerhalb und in einem konstanten ersten Abstand AB1 verlaufenden imaginären inneren Abstandslinie begrenzt wird, mindestens 10%, insbesondere mindestens 15%, insbesondere mindestens 20%, insbesondere mindestens 25%, weiter insbesondere mindestens 30% einer Größe der Fläche A1 beträgt, wobei als die Fläche A1 die durch die Randumfangslinie des Kennzeichnungsmittels eingeschriebene Fläche definiert ist. Vorzugsweise beträgt die Fläche A3 höchstens 50%, weiter insbesondere höchstens 45%, weiter insbesondere höchstens 40%, weiter insbesondere höchstens 35% einer Größe der Fläche A1.

Weiter vorzugsweise beträgt der Flächenanteil des zweiten Kennzeichens an der Fläche A3 höchstens 10%, insbesondere höchstens 7%, weiter insbesondere höchstens 5%, insbesondere höchstens 2% beträgt. Die Bestimmung soll hierbei im vollständig ausgebreiteten Zustand der Inkontinenzvorlage erfolgen. Die Größen der Flächen werde jeweils in mm² vermessen.

Hierdurch wird eine gute visuelle Wahrnehmbarkeit des ersten als auch des zweiten Kennzeichens ermöglicht.

Durch eine derartige Gestaltung einer Fläche A3, die innerhalb der Fläche A1 liegt, wird der visuelle Eindruck einer zumindest dickeren/breiteren Umrandungsfläche erreicht, die sich in der Art eines Randes um das zweite Kennzeichen erstreckt, wodurch die visuelle Sichtbarkeit und Erkennbarkeit verbessert wird, insbesondere auch bei ansonsten nur schwerlesbaren alphanumerischen Zeichen als zweites Kennzeichen.

Insbesondere kann vorgesehen sein, dass zwischen der Randumfangslinie des Kennzeichnungsmittels, also einer Randumfangslinie der visuell wahrnehmbaren farbigen Zone des ersten Kennzeichens, und einer in einem konstanten ersten Abstand AB1 verlaufenden imaginären inneren Abstandslinie, die einen Abstand von mindestens 2 mm, weiter insbesondere 3 mm, weiter insbesondere mindestens 4 mm und weiter insbesondere mindestens 5 mm aufweist, zumindest abschnittsweise, insbesondere vollumfänglich keine Aussparungen vorgesehen sind.

Um diese visuelle Erkennbarkeit und das visuell wahrnehmbare Abheben des Kennzeichnungsmittels bzw. der farbigen Zone des ersten Kennzeichens von den zweiten Bereichen des Trägerflachbahnmaterials, insbesondere des Deckblatt-Materials und/oder des Backsheet-Materials und als solche das Kennzeichnungsmittel umgebend weiter zu verbessern, kann vorgesehen sein, dass die Größe einer Fläche A4, welche von einer Randumfangslinie des Kennzeichnungsmittels und einer davon außerhalb und in einem konstanten zweiten Abstand AB2 verlaufenden imaginären Abstandslinie begrenzt wird, mindestens 30%, insbesondere mindestens 40%, insbesondere mindestens 50%, insbesondere mindestens 60%, weiter insbesondere mindestens 70% der Größe einer Fläche A1 beträgt, wobei als die Fläche A1 die durch die Randumfangslinie des Kennzeichnungsmittels eingeschriebene Fläche definiert ist, wobei vorzugsweise mindestens 90%, insbesondere mindestens 93%, insbesondere mindestens 95% der Fläche A4 frei von weiteren farbigen Zonen, insbesondere als Beschichtungen und/oder Bedruckungen und/oder Änderungen der dem Trägerflachbahnmaterial, insbesondere dem Deckblatt-Material und/oder Backsheet-Material zu eigenen optischen visuellen und/oder haptischen Beschaffenheit sind.

Die Größen der Flächen werden jeweils in mm² vermessen. Die Bestimmung soll hierbei im vollständig plan ausgebreiteten Zustand der Inkontinenzvorlage erfolgen.

Darüber hinaus kann insbesondere vorgesehen sein, dass zwischen der Randumfangslinie des Kennzeichnungsmittels, also einer Randumfangslinie der farbigen Zone des ersten Kennzeichens, und einer in einem konstanten zweiten Abstand AB2 verlaufenden imaginären äußeren Abstandslinie, die einen Abstand von mindestens 2 mm, weiter insbesondere mindestens 3 mm, weiter insbesondere mindestens 4 mm und weiter insbesondere mindestens 5 mm, weiter insbesondere mindestens 8 mm, weitere insbesondere mindestens 10 mm aufweist, eine innerhalb dieses zweiten Abstands A2 begrenzten Fläche zumindest abschnittsweise, insbesondere vollumfänglich keine farbige Zone, wie z.B. in Form einer Bedruckung vorgesehen ist.

Weiter kann insbesondere vorgesehen sein, dass das Kennzeichnungsmittel eine durch eine Randumfangslinie eingeschriebene Fläche A1 überfängt, und das zweite Kennzeichen einen Flächenanteil von höchstens 50%, insbesondere höchstens 40%, weiter insbesondere höchstens 30%, weiter insbesondere höchstens 20%, und insbesondere wenigstens 3 %, insbesondere wenigstens 5 %, insbesondere wenigstens 8 % der vom Kennzeichnungsmittel überfangenen Fläche A1 einnimmt. Hierdurch bleibt die visuelle Sichtbarkeit des ersten Kennzeichens bestehen.

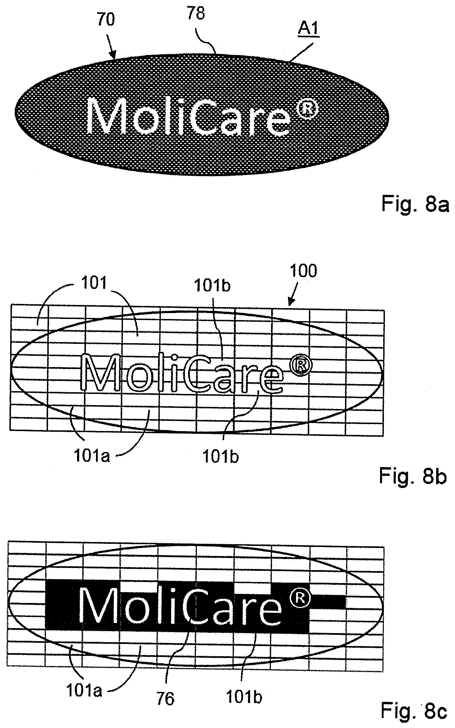

Für die Erkennbarkeit einerseits aber auch die Diskretheit andererseits ist insbesondere eine weitgehend ausgewogene Verteilung des zweiten Kennzeichens innerhalb des ersten Kennzeichens von Vorteil. Die Bestimmung des Verteilungsgrad ist dabei beispielhaft anhand der Figuren 8a-8c erläutert.

Es kann dabei bevorzugt vorgesehen sein, dass wenn das Kennzeichnungsmittel mit einer Randumfangslinie in seinen jeweils äußersten Punkten von einem imaginären Rechteck tangierend eingeschrieben ist, wobei das Rechteck bezüglich seiner Kanten in Längs- und Querrichtung des Inkontinenzartikels angeordnet ist und dabei sowohl in Längsrichtung als auch in Querrichtung in jeweils 10 gleich große Unterrechtecke unterteilt ist, von einer Anzahl N1 an Unterrechtecken, welche vollständig oder zumindest bereichsweise innerhalb der Randumfangslinie lokalisiert sind und davon einer Anzahl N2 derjenigen Unterrechtecken, die zumindest bereichsweise sowohl das erste Kennzeichen als auch das zweite Kennzeichen aufweisen, ein Verteilungsgrad von N2/N1*100% von mindestens 10 %, insbesondere mindestens 20 %, insbesondere mindestens 25%, insbesondere mindestens 30%, insbesondere mindestens 35% vorgesehen ist. Weiter vorzugsweise ist ein Verteilungsgrad von insbesondere höchstens 60%, weiter insbesondere höchstens 55 %, weiter insbesondere höchstens 50%, weiter insbesondere höchstens 45% vorgesehen.

Es ist insbesondere vorgesehen, dass das erste Kennzeichen im ersten Bereich eine erste Farbe aufweist und ein an das erste Kennzeichen direkt angrenzende zweite Bereich eine davon unterscheidbare dritte Farbe aufweist. Weiter insbesondere ist vorgesehen, dass bei einem ein zweites Kennzeichen umfassenden Kennzeichnungsmittel, das erste Kennzeichen eine erste Farbe aufweist und das zweite Kennzeichen eine davon unterscheidbare zweite Farbe aufweist. Insbesondere ist vorgesehen, dass die zweite Farbe und die dritte Farbe annähernd gleich sind.

Insbesondere werden die Farben nach CIEL*a*b* vermessen. Die Messung und Berechnung ist nachfolgend beschrieben.

Insbesondere ist vorgesehen, dass das erste Kennzeichen im ersten Bereich eine erste Farbe aufweist und ein an das erste Kennzeichen direkt angrenzende zweite Bereich eine davon unterscheidbare dritte Farbe aufweist, wobei die Farbe, jeweils auf der Innenseite und/oder jeweils auf der Außenseite nach CIEL*a*b* vermessen ist, und ein nach CIEL*a*b* berechneter Farbabstand zwischen der ersten und der dritten Farbe, dargestellt als Delta E (ΔE*), besteht.

Insbesondere beträgt der nach CIEL*a*b* berechnete Farbabstand zwischen der ersten und der dritten Farbe dargestellt als Delta E (ΔE*) 10 bis 55, vorzugsweise 15 bis 55, weiter vorzugsweise 20 bis 55, weiter vorzugsweise 35 bis 55.

Auf diese Weise wird ein Kennzeichnungsmittel bereitgestellt, dessen farbige Zone des ersten Kennzeichens von dem dieses erste Kennzeichen umgebenden Bereichs gut visuell unterscheidbar ist und dem Kennzeichnungsmittel somit eine gute Wahrnehmbarkeit und damit eine Signalwirkung zukommen kann.

In einer bevorzugten Ausführungsform des Kennzeichnungsmittels, das ein zweites Kennzeichen in Form von Aussparungen innerhalb des ersten Kennzeichens aufweist, ist es insbesondere vorgesehen, dass das erste Kennzeichen im ersten Bereich eine erste Farbe aufweist und das zweite Kennzeichen eine davon unterscheidbare zweite Farbe aufweist, wobei die Farbe, jeweils auf der Innenseite und/oder jeweils auf der Außenseite nach CIEL*a*b* vermessen ist, ein nach CIEL*a*b* berechneter Farbabstand zwischen der ersten und der zweiten Farbe, dargestellt als Delta E (ΔE*), besteht.

Insbesondere beträgt der nach CIEL*a*b* berechnete Farbabstand zwischen der ersten und der zweiten Farbe dargestellt als Delta E (ΔE*) 10 bis 55, vorzugsweise 15 bis 55, weiter vorzugsweise 20 bis 55, weiter vorzugsweise 35 bis 55.

Durch ein sich vom zweiten Kennzeichen abhebendes erste Kennzeichen, der farbigen Zone, wird ein Kontrast geschaffen, der es ermöglicht, die Wahrnehmbarkeit und/oder Lesbarkeit des zweiten Kennzeichens, sogar in einer vergleichsweise klein dimensionierten Ausbildung, zu verbessern.

Insbesondere ist der Farbabstand zwischen erster und zweiter Farbe und der Farbabstand zwischen erster und dritter Farbe im Wesentlichen gleich.

Da das zweite Kennzeichen, in Form von Aussparungen innerhalb der farbigen Zone gebildet ist, und vorzugsweise ohne weitere Oberflächenbehandlung von der Beschaffenheit und/oder Farbe des Materials des dieses erste Kennzeichen umgebenden Bereichs, nämlich zweiten Bereichs gebildet wird, bestimmt dessen Beschaffenheit/Farbe auch den Wert des zweiten Kennzeichens.

Insbesondere auf der Außenseite beträgt ein nach CIEL*a*b* berechneter Farbabstand zwischen der ersten und der zweiten Farbe und/oder zwischen der ersten und dritten Farbe, als Delta E (ΔE*) insbesondere 15 bis 55, vorzugsweise 20 bis 55, weiter vorzugsweise 35 bis 55.

Insbesondere kann ein nach CIEL*a*b* berechneter Farbabstand auf der Außenseite zwischen der ersten und der zweiten Farbe und/oder zwischen der ersten und dritten Farbe größer sein als der auf der Innenseite nach CIEL*a*b* berechnete Farbabstand zwischen der jeweiligen ersten und der zweiten Farbe und/oder der jeweiligen ersten und dritten Farbe.

Dabei unterscheiden sich die jeweiligen Farbabstände von Außenseite und Innenseite, berechnet als Differenz zwischen dem jeweiligen Farbabstand der Außenseite und Innenseite, um einen Betrag von höchstens 20, weiter insbesondere höchstens 18, weiter insbesondere höchstens 16, weiter insbesondere höchstens 14, weiter insbesondere höchstens 12, weiter insbesondere höchstens 10.

Insbesondere kann ein auf der Innenseite nach CIEL*a*b* berechneter Farbabstand zwischen der ersten und der zweiten Farbe und/oder zwischen der ersten und dritten Farbe 15- 40, vorzugsweise 20 - 40 betragen.

Auch in dieser Ausführung ist ein Kontrast innerhalb des Kennzeichnungsmittels zwischen erstem Kennzeichen und dem darin umschlossenen zweitem Kennzeichen und/oder ein Kontrast zwischen der dem Kennzeichnungsmittel zugehörigen farbigen Zone des ersten Kennzeichens und dem das Kennzeichnungsmittel umgebenden Bereichs durchaus vorhanden, um auch auf der Produktinnenseite ein Kennzeichnungsmittel mit Wahrnehmungskraft bereitzustellen.

Der auf der Außenseite zwischen dem ersten und zweiten Kennzeichen im Vergleich zur Innenseite vorzugsweise größere Farbabstand deutet auf einen höheren Kontrast auf der Produktaußenseite hin, was vorteilhaft für die auf der Produktaußenseite durchaus gewünscht höhere Attraktion des Kennzeichnungsmittels ist. Zumal auf der Außenseite oftmals auch andere Markierungen vorhanden sein können, die der Wahrnehmung des Kennzeichnungsmittels in Konkurrenz treten können. Hingegen braucht es auf der Produktinnenseite zumeist keine übersteigerte Attraktion, bei einer auf der Innenseite von einer flächigen Erstreckung des Saugkörpers dominierten Aufsicht, die dabei zumeist einfarbig einheitlich ausgestaltet ist. Dennoch ist das Kennzeichnungsmittel visuell wahrnehmbar und gegenüber der sonstigen Fläche visuell erkennbar.

Vorzugsweise ist vorgesehen, dass in dem ersten Bereich, in dem das Kennzeichnungsmittel und damit die das erste Kennzeichen bildende farbige Zone angebracht ist, zwischen dem Kennzeichnungsmittel und der jeweiligen Außenseite und Innenseite vorzugsweise nur eine geringe Anzahl an Materiallagen vorgesehen ist, ganz besonders bevorzugt unterscheidet sich die Anzahl der Lagen zwischen dem Kennzeichnungsmittel zur Innenseite und der Anzahl der Lagen zwischen dem Kennzeichnungsmittel zur Außenseite höchstens um 2 Lagen, insbesondere höchstens um 1 Lage. Eine entsprechende Verteilung der Lagen ist vorteilhaft auch in dem diesen ersten Bereich umgebenden zweiten Bereich vorgesehen. Dies unterstützt die visuelle Erkennbarkeit des Kennzeichnungsmittels auf der Außenseite und Innenseite.

Vorzugsweise ist vorgesehen, dass die innerhalb des ersten Bereiches des ersten Kennzeichens vorhandenen Materiallagen eine jeweilige Opazität nicht übersteigen, um eine visuelle Wahrnehmung des Kennzeichnungsmittels sowohl auf der Innenseite als auch auf der Außenseite zu ermöglichen.

Die Opazität ist ein Maß für die Lichtundurchlässigkeit einer Materiallage.

Bevorzugt weisen im Bereich des Kennzeichnungsmittels vorgesehene Materiallagen eine Opazität von höchstens 60%, weiter vorzugsweise höchstens 50%, weiter vorzugsweise höchstens 40%, weiter vorzugsweise höchstens 30% auf. Insbesondere bevorzugt weist das Deckblatt-Material eine Opazität von höchstens 60%, weiter vorzugsweise höchstens 50%, weiter vorzugsweise höchstens 40%, weiter vorzugsweise höchstens 30% auf. Weiter vorzugweise weist das Backsheet-Material eine Opazität von höchstens 70%, weiter vorzugsweise höchstens 60%, weiter vorzugsweise höchstens 50%, weiter vorzugsweise von 40%, weiter vorzugsweise von 30% auf.

Dabei erfolgt die Farbmessung nach CIEL*a*b* wie folgt:
**Farbmessung nach dem CIEL*a*b*-Farbraum und daraus abgeleitete Farbabstände, sogenannte ΔE*-Werte:**
Die Farbmessung nach L*a*b*, auch genannt CIEL*a*b*-Farbraum, beschreibt und definiert Farben geräteunabhängig. Der CIEL*a*b*-Farbraum ist genormt in der DIN EN ISO 11664-4: 2012-06 Teil 4.

Der CIEL*a*b*-Farbraum basiert im Wesentlichen auf der Darstellung einer Farbe innerhalb eines Koordinatensystems mit drei Achsen. Die Achse L* repräsentiert die Helligkeit zwischen 0 (schwarz) - 100 (weiß); die a*-Achse gibt den Grün- oder Rotanteil wider, mit negativen Werten für Grün und positiven Werten für Rot. Die b*-Achse gibt den Blau-oder Gelb-Anteil wider, dabei mit negativen Werten für Blau und positive Werte für Gelb.

### Prüfgerät

Für die Farbmessungen wird als Prüfgerät ein Spektralphotometer eingesetzt, wie z.B. das Spectro-guide sphere gloss von BYK-Gardner.

### Probenvorbereitung:

Die Messprobe bzw. den Artikel auf einer ebenen Fläche flach ausbreiten. Für die Probengröße ist letztlich die von der Messfeldbegrenzung des Prüfgerätes vorgegebene Messfläche zu beachten.

Die Messungen wird im plan ausgebreiteten Zustand der Messprobe bzw. des Artikels vorgenommen.

Die Messungen werden an der Produktinnenseite und/oder Produktaußenseite des Kennzeichnungsmittels aufweisenden hinteren Produktendabschnitts vorgenommen.

Die Produktinnenseite versteht sich dabei als die für den Anwendungszustand körperzugewandte Oberseite und die Produktaußenseite als im Anwendungszustand körperabgewandte Oberseite des Inkontinenzartikels.

Die Messungen werden direkt am Produkt vorgenommen. Um die vom Kennzeichnungsmittel, bzw. vom ersten und/oder zweiten Kennzeichen in der Anwendung vermittelten Farbreize zu analysieren, werden die Farbmessungen an der Produktinnenseite und/oder Produktaußenseite im das Kennzeichnungsmittel aufweisenden Bereich und/oder in einem an das erste Kennzeichen direkt angrenzenden Bereich vorgenommen, unabhängig davon, ob die farbige Zone tatsächlich direkt auf einer die Produktinnenseite oder Produktaußenseite bildenden Materiallage, wie beispielsweise Oberseite des Deckblatts-Materials/Backsheet-Materials auf/angebracht ist oder nicht.

Letzteren Falls wird auf die auf der Produktinnenseite oder Produktaußenseite erkennbaren ersten und zweiten Kennzeichen abgestellt.

Die zu vermessende Oberseite der Messprobe ist dabei dem Prüfgerät und dessen optischen Messfeldbereichen zugewandt angeordnet.

### Kalibrieren:

Das Prüfgerät ist nach den Herstellervorgaben und dafür vorgesehenen Standards kalibriert.

### Messvorgang:

Für die hierbei vorzunehmenden Messungen wird eine standardisierte weiße Testkarte als Referenzfläche unterhalb der Messprobe im Bereich des vorgesehenen Messfeldes gelegt, so dass die jeweiligen Farbmessungen stets gegenüber einer Standardfläche durchgeführt werden.

Als Referenzfläche werden die für die Opazitätsmessung vorgesehene "opacity charts" von BYK-Gardner, dabei die Karte #2810 eingesetzt (erhältlich bei BYK-Gardner GmbH, Geretsried, Deutschland).

Diese weiße Referenzflächen-Testkarte weist einen L*a*b*-Wert von
L* von 92,12 ± 0,79
a* von -0,95 ± 0,14
b* von 3,69 ± 0,42 auf,
dabei als Mittelwert ± 6 sigma (Standardabweichung)

Für den Messvorgang wird der Messkopf des Gerätes senkrecht und ohne zusätzlichen Druck auf die vorbereitete Messprobe und die gewünschte zu messende Oberseite der Messprobe aufgesetzt. Dafür wird, je nach Konstruktion des Prüfgerätes, zuerst mit einer Komponente die eine Messfeldbegrenzung aufweist (sogenannter Probenbeobachter), die für die Messung relevante Stelle der Messprobe auf der Messprobe festgelegt. Diesem Messfeld gegenüber wird dann das Prüfgerät mit seiner Messöffnung aufgesetzt.

Für das Prüfverfahren und den Messvorgang werden am Prüfgerät als Einstellungen vorgenommen:
- Skala: CIEL*a*b*
- Index: ΔE*
- Lichtart: D65
- Winkel: 10°
- Messöffnung: 11 mm
- im Falle von dazu schmaleren Messbereichen, ist ein Prüfgerät mit einer entsprechend schmäleren Messöffnung zu wählen

Es werden von einer Messprobe n = 3 Messungen vorgenommen.

Für die Farbmessung für das erste Kennzeichen ist vorrangig ein jeweiliger Bereich innerhalb der farbigen Zone 73 des ersten Kennzeichens innerhalb des Kennzeichnungsmittels heranzuziehen. Sollte kein genügend großer Bereich zur Verfügung zu stehen, so wird ein Messfeld mit dem größtmöglichen Anteil an erstem Kennzeichen, also an farbiger Zone 73 herangezogen.

Für die Farbmessung für das zweite Kennzeichen werden bei ausreichend großen Aussparungen 76 die Farbmessungen innerhalb der Aussparungen 76 vorgenommen. Andernfalls werden die Farbmessungen in einem außerhalb der Randumfangslinie 78 des ersten Kennzeichens direkt angrenzenden Bereich der die Außenseite und/oder Innenseite bildenden Materiallage, wie beispielsweise des Deckblattmaterials oder Backsheet-Materials, durchgeführt und zwar dort wo sich das die Außenseite bzw. Innenseite bildende Material, wie beispielsweise das Deckblattmaterial oder Backsheet-Material, visuell wahrnehmbar nicht von dem Material, wie beispielsweise vom Deckblattmaterial oder Backsheet-Material, in den Aussparungen unterscheidet. Dabei wird die Messung an verschiedenen Stellen durchgeführt.

### Auswertung:

Aus den Messwerten wird der Mittelwert ermittelt.

Die Messwerte und der Mittelwert werden auf eine Dezimale nach dem Komma angegeben.

Die Farbtöne werden als L*, a*, b* Wert angegeben. Der Wert ist dimensionslos.

Für den Vergleich von Farbmessungen an verschiedenen Bereichen bzw. verschiedenen Messproben wird der Farbabstand bei Farbmessungen nach dem CIEL*a*b*-Farbraum als Delta-E (ΔE*) wie folgt berechnet:
- für die jeweilige Differenz von jeder der drei Achsen des CIEL*a*b*-Farbraums das Quadrat berechnen
- davon die Summe und über diese Summe die Wurzel berechnen: $ΔE * = {\left[{\left(ΔL*\right)}^{2}+{\left(Δa*\right)}^{2}+{\left(Δb*\right)}^{2}\right]}^{0 , 5}$

Der Wert ist dimensionslos.

Dabei erfolgt die Messung der Opazität wie folgt:

### Opazität

Die Opazität ist ein Maß für die Lichtundurchlässigkeit einer Materialschicht, oder Materiallagen-Kombinationen und damit ein Maß für die Fähigkeit eines Materials, den Hintergrund dahinter zu verdecken. Die Opazität wird dabei bestimmt über das Verhältnis aus bei einem schwarzen Hintergrund für ein Material erhaltene Reflexionsvermögen und für das gleiche Material bei einem weißen Hintergrund erhaltene Reflexionsvermögen. Ein höherer Opazitätswert zeigt dabei einen höheren Grad an Lichtblockierung durch das Material an.

### Prüfgerät:

Für die Opazitätsmessungen wird als Prüfgerät ein Spektralphotometer eingesetzt, wie z.B. das Spectro-guide sphere gloss von BYK-Gardner.

### Kalibrieren:

Das Prüfgerät ist nach den Herstellervorgaben und dafür vorgesehenen Standards kalibriert.

### Messvorgang:

Für die Opazitätsmessung werden die vom Hersteller vorgegebenen Schwarz/Weiß-Opazitätskarten eingesetzt. Von den vorgesehenen "opacity charts" von BYK-Gardner, wird dabei die Karte #2810 eingesetzt (erhältlich bei BYK-Gardner GmbH, Geretsried, Deutschland).

Für das Prüfverfahren und den Messvorgang werden am Prüfgerät als Einstellungen vorgenommen:
- Skala: CIEL*a*b*
- Index: Opazität
- Lichtart: D65
- Winkel: 10°
- Messöffnung: 11 mm
- im Falle von dazu schmaleren Messbereichen, ist ein Prüfgerät mit einer entsprechend schmäleren Messöffnung zu wählen

Es werden von einer Messprobe n = 3 Messungen vorgenommen.

Für den Messvorgang wird der Messkopf des Gerätes senkrecht und ohne zusätzlichen Druck auf die vorbereitete Messprobe und die gewünschte zu messende Oberseite der Messprobe aufgesetzt. Es wird der Probenbeobachter verwendet um Einflüsse durch unterschiedliche Lichtbedingungen zu vermeiden. Die Messprobe sollte dabei das Messfeld des Prüfgerätes vollständig überdecken.

Die Opazität wird kann dabei im Differenzmodus zwischen der Messung über dem schwarzen Untergrund und der anschließenden Messung über weißem Untergrund bestimmt werden.

Das für die Opazität zu vermessende Material (Messprobe) wird dazu auf die S/W-Opazitätskarte gelegt: Dabei wird die Messprobe ohne Kontamination des vorgesehenen Messfeldes zunächst auf das schwarze Feld der S/W-Opazitätskarte gelegt. Der Messvorgang mittels des Prüfgerätes wird gestartet, die abgeschlossene Messung durch das Prüfgerät wird mit einem Signalton quittiert. Es erfolgt ein Abgleich nach dieser ersten Messung, indem die über schwarzem Untergrund gemessene Opazität auf Null gesetzt wird. Dann wird das gleiche Material, mit dem selben Probenausschnitt der vorangehenden Messung, auf das weiße Feld der S/W-Opazitätskarte positioniert, und dort wird ebenso die Messung mittels Prüfgerät durchgeführt. Der Messwert im weißen Feld wird bereits als Differenzwert ausgegeben und stellt den ermittelten Opazitätswert der Messprobe dar, mit der Angabe des Wertes in %.

Aus den Messwerten wird der Mittelwert ermittelt. Die Angabe erfolgt in Prozent.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen der erfindungsgemäßen Inkontinenzvorlage.

In der Zeichnung zeigen:
- Figur 1: eine Draufsicht auf eine erfindungsgemäße Inkontinenzvorlage in einem flach gelegten und eben ausgebreiteten Zustand;
- Figur 2: eine Gestaltung einer Inkontinenzvorlage ähnlich dem in Figur 1 Gezeigten,
- Figuren 3a, 3b und 3c: eine Schnittdarstellung in Längsrichtung durch den hinteren Produktendabschnitt der Inkontinenzvorlage in einer schematischen Darstellung,
- Figuren 4a, 4b und 4c: eine vergleichende Betrachtung eines mit dem Kennzeichnungsmittel im hinteren Produktendabschnitt eingeschriebenen Bereichs mit einem analogen Bereich im vorderen Produktendabschnitt,
- Figuren 5a, 5b, 5c und 5d: eine Inkontinenzvorlage mit vorgesehenen Faltungsabschnitten und Schnittdarstellungen von gefalteten Konfigurationen,
- Figur 6: schematisch eine vergrößerte Darstellung einer bevorzugten Ausführungsform des Kennzeichnungsmittels,
- Figuren 7a, 7b, 7c, 7d: das Kennzeichnungsmittel mit der Bestimmung eines inneren und eines äußeren Abstands ausgehend von der Randumfangslinie,
- Figuren 8a, 8b und 8c: eine Bestimmung des Anteils des ersten und zweiten Kennzeichens an der Fläche des Kennzeichnungsmittels,
- Figur 9: schematisch eine Bestimmung einer funktional zusammenhängenden Anordnung von Sub-Kennzeichnungsmittel zu einem Kennzeichnungsmittel und
- Figur 10: schematisch ein Kennzeichnungsmittel bestehend aus zwei Sub-Kennzeichnungsmittel

Die Figuren zeigen eine insgesamt mit dem Bezugszeichen 2 bezeichnete wegwerfbare absorbierende Inkontinenzvorlage für die Aufnahme von im Wesentlichen flüssigen Körperausscheidungen.

Die Inkontinenzvorlage 2 weist in Längsrichtung 4 über die Produktlänge L1 einen hinteren Produktabschnitt 14, einen mittleren Produktabschnitt 15 und einen vorderen Produktendabschnitt 16 auf, die dabei jeweils ein Drittel der Produktlänge L1 erfassen.

Die Inkontinenzvorlage 2 mit einer Produktlänge L1 von vorzugsweise 450 - 800 mm und mit einer Produktbreite B1 von vorzugsweise 200 - 450 mm, somit vor allem als eine Erwachsenen-Inkontinenzvorlage vorgesehen, weist entlang ihrer Längsrichtung unterschiedliche Eigenschaften auf. Eine mittels der Quermittelachse 10 abgrenzte hintere Produktlängshälfte 17 und vordere Produktlängshälfte 18 sind bezogen auf die Quermittelachse zumindest hinsichtlich einer Eigenschaft nicht spiegelsymmetrisch. Unterschiede können dabei insbesondere Eigenschaften, wie die Fläche des Produkts, die Fläche des Saugkörpers, insbesondere auch den Lagenaufbau des Saugkörpers, die Saugleistung des Saugkörpers und die chemische Zusammensetzung des Saugkörpers betreffen. Die damit verbundenen unterschiedlichen Funktionsbereiche innerhalb der Inkontinenzvorlage bedürfen somit der richtigen Anordnung der Inkontinenzvorlage am Träger.

Der Abstand der Konturlinie 13 der Inkontinenzvorlage beidseits der Längsmittelachse 8 kann über die Längsrichtung 4 variabel sein. So weist der hintere Produktabschnitt 14 eine maximale Querstreckung B2 und der vordere Produktabschnitt 16 eine maximale Quererstreckung B3 auf, die sich bei der Betrachtung des Quotienten B2/B3 oftmals auch nur gering voneinander unterscheiden können, so mit einem Wert von vorzugsweise ≥ 1 und ≤ 1,25.

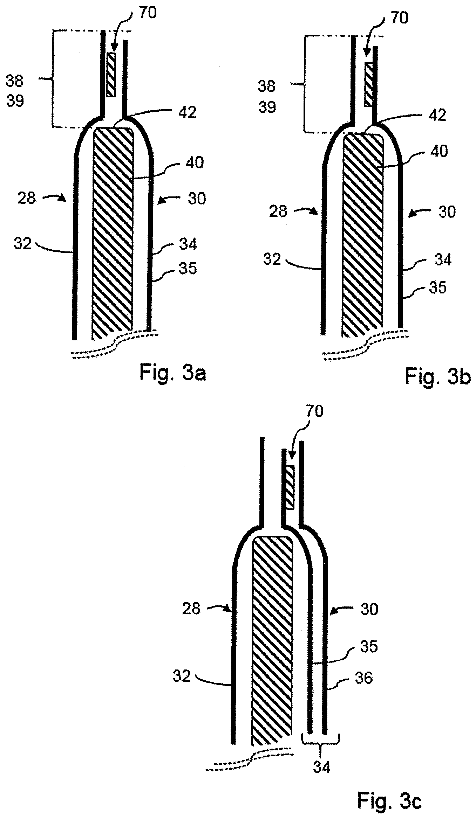

Die Inkontinenzvorlage 2 mit einer Innenseite 28, die im Gebrauch dem Körper des Trägers zugewandte Oberseite, und mit einer Außenseite 30, die im Gebrauch vom Körper des Trägers abgewandte Oberseite, umfasst zumindest ein Deckblatt-Material 32, ein Backsheet-Material 34 und einen dazwischen angeordneten Saugkörper 40. Im vorliegenden Ausführungsbeispiel erstreckt sich das Deckblatt-Material 32 und das Backsheet-Material 34 in einen Bereich 39 außerhalb der den Saugkörper 40 begrenzenden Konturlinie 42 unter Ausbildung eines sogenannten Überhang 38, wie weiter auch schematisch in den Figuren 3a, 3b, 3c als Schnittansichten durch einen hinteren Produktendabschnitt ersichtlich ist.

Innerhalb eines hinteren Produktendabschnitts 20 und innerhalb des vorderen Produktendabschnitt 24, die jeweils ein Zehntel der Produktlänge L1 erfassen, ist ein hinterer Quernahtabschnitt 46 bzw. entsprechend ein vorderer Quernahtabschnitt 48 vorgesehen, wobei der Quernahtabschnitt den jeweiligen Bereich zwischen der den Saugkörper 40 begrenzenden Konturlinie 42 und der die Inkontinenzvorlage begrenzenden Konturlinie 13 erfasst.

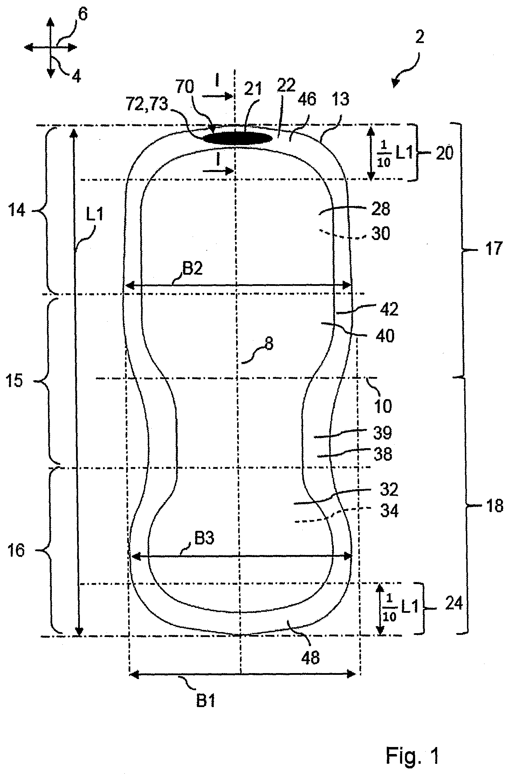

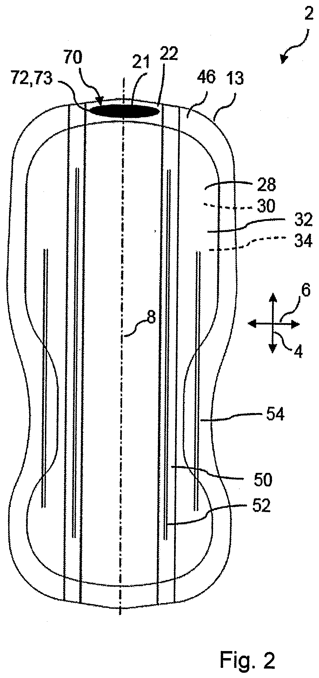

Figur 2 zeigt nun eine Gestaltung einer Inkontinenzvorlage 2 ähnlich dem in Figur 1 Gezeigten, wobei hier zusätzlich beidseits in Längsrichtung 4 verlaufende Schrittelastifizierungsmittel 54 vorgesehen sind, die in Anwendung zur Ausbildung einer bootförmigen Konfiguration der Inkontinenzvorlage beitragen. Einwärts der Schrittleastifzierungsmittel 54 sind auf der Innenseite 28 beidseits in Längsrichtung 4 erstreckende Cuffelemente 50 vorgesehen, die ebenso mit Elastifizierungsmittel 52 ausgestattet sind und eine Auslaufsperre für Flüssigkeiten darstellen.

Die Inkontinenzvorlage 2 weist, wie in Figur 1 und 2 gezeigt, ein Kennzeichnungsmittel 70 auf, das im hinteren Produktendabschnitt 20, dabei insbesondere vollumfänglich innerhalb des hinteren Quernahtabschnitts 46 angeordnet ist.

Der hintere Produktendabschnitt 20, insbesondere der hintere Quernahtabschnitt 46 umfasst zumindest einen ersten Bereich 21 und einen zweiten Bereich 22, wobei in dem ersten Bereich 21 das Kennzeichnungsmittel 70 vorgesehen ist. Das Kennzeichnungsmittel weist dabei ein erstes Kennzeichen 72 auf, das durch die flächenhaft erstreckte farbige Zone 73 gebildet ist und im vorliegenden Fall die Information transportiert, wo die Rückseite der Inkontinenzvorlage 2 ist. Darüber hinaus wird insbesondere durch die Wahl der Farbe eine Information darüber generiert, dass es sich um eine Inkontinenzvorlage von definierter Saugstärke handelt.

Im hinteren Quernahtabschnitt 46 sind das Deckblatt-Material 32 und das Backsheet-Material 34 vorzugsweise adhäsiv miteinander verbunden. Das Deckblatt-Material 32 umfasst vorzugsweise ein Vliesmaterial und das Backsheet-Material 34 umfasst zumindest eine Folienlage 35 und insbesondere bevorzugt zusätzlich eine zur Außenseite 30 angeordnete Vlieslage 36 unter Ausbildung eines sogenannten textilen Backsheets. Das Kennzeichnungsmittel 70 kann als Beschichtung auf einer der Außenseite 30 zugewandten Oberseite des Deckblatt-Materials 32 (Figur 3a) oder insbesondere auf einer der Innenseite 28 zugewandten Oberseite einer Folienlage 35 des Backsheet-Materials 34 (Figur 3b) angebracht sein. Bei einem textilen Backsheet (Figur 3c) ist bevorzugt auf der der Außenseite 30 zugewandten Oberseite der Folienlage 35 eine farbige Zone 73 vorgesehen, die das Kennzeichnungsmittel 70 bildet. Auf diese Weise wird erreicht, dass kein Abrieb der farbigen Zone erfolgen kann.

Die farbige Zone 73 des ersten Kennzeichens 72 des Kennzeichnungsmittel 70, wie in Figur 1 und 2 dargestellt, ist hier in Form eines Farbaufdrucks auf eine der im hinteren Quernahtabschnitt 46 vorgesehenen Trägerflachbahnmaterialien, wie eben dem Deckblatt-Material 32 oder dem Backsheet-Material 34 aufgebracht, dabei vorzugsweise in einer der Anordnung wie in den Figuren 3a - 3c gezeigt.

Das Kennzeichnungsmittel 70 ist dabei von der Innenseite 28 und von der Außenseite 30 der Inkontinenzvorlage visuell erkennbar.

Unterstützt wird die visuelle Erkennbarkeit des Kennzeichnungsmittels auf der Außenseite und Innenseite dadurch, dass zwischen dem Kennzeichnungsmittel und der jeweiligen Außenseite und Innenseite in dem ersten Bereich 21, in dem das Kennzeichnungsmittel angeordnet ist, vorzugsweise nur eine geringe Anzahl an Materiallagen vorgesehen ist, ganz besonders bevorzugt unterscheiden sich die Anzahl der Lagen zwischen Kennzeichnungsmittel zur Innenseite und der Anzahl der Lagen zwischen Kennzeichnungsmittel zur Außenseite höchstens um 2, insbesondere höchstens um 1 Lage.

In Figur 3a ist das Kennzeichnungsmittel 70 sowohl von der Innenseite 28 als auch von der Außenseite 30 von einer Flachbahnmateriallage, nämlich dem Deckblattmaterial 32 zur Innenseite und der Folie 35 zu Außenseite überdeckt. Entsprechendes mit nur einer Flachbahnmateriallage zwischen Kennzeichnungsmittel 70 und der Außenseite bzw. der Innenseite zeigt die Figur 3b. In Figur 3c ist das Kennzeichnungsmittel 70 ausgehend von der Innenseite 28 von der Lage des Deckblatt-Materials 32 und der Folienlage 35 des textilem Backsheet-Materials 34 überdeckt und ausgehend von der Außenseite 30 von der Vlieslage 36.

Das Deckblatt-Material, vorzugsweise aus Vliesstoffmaterial mit einem Flächengewicht von 5 - 20 g/m² und das Backsheet-Material mit einem Flächengewicht von vorzugsweise 9-30 g/m² weisen jeweils ein Opazität von höchstens 60%, vorzugsweise von höchstens 50%, insbesondere höchstens 40% auf, was die visuelle Erkennbarkeit des Kennzeichnungsmittels auf der Innenseite und Außenseite unterstützt.

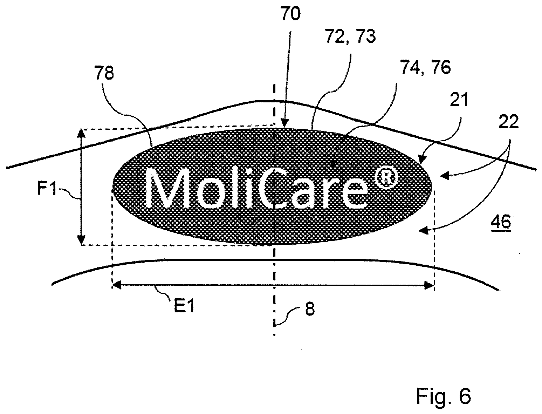

In einer bevorzugten Ausführungsform des Kennzeichnungsmittels 70, wie in Figur 6 vergrößert schematisch dargestellt ist, sind in dem Kennzeichnungsmittel 70 bzw. in der farbigen Zone 73 des ersten Kennzeichens 72 Ausnehmungen 76 vorgesehen, die mit einem gewissen Abstand zur Randumfangslinie 78 des Kennzeichnungsmittels 70 angeordnet sind.

Die Ausnehmungen 76 bilden ein zweites Kennzeichen 74, das eine weitere Information transportiert, nämlich vorzugweise über die Vorsehung von alphanumerischen Zeichen, den Hinweis über einen Hersteller bzw. seiner Marke.

Mit der Ausgestaltung der Ausnehmungen 76 als alphanumerische Zeichen, dessen informativen Inhalt und Kontext der Anwender durch intuitive Lesen erfasst, wird die Aufmerksamkeit eines Anwenders noch mehr auf das Kennzeichnungsmittel 70 gelenkt und damit die Wahrnehmungskraft des Kennzeichnungsmittels unterstützt.

Dabei bilden die Aussparungen 76 Bereiche in der farbigen Zone 73, in denen insbesondere die visuell wahrnehmbare Beschaffenheit des Materials derjenigen visuell wahrnehmbaren Beschaffenheit des Materials des zweiten Bereichs 22 im hinteren Quernahtabschnitt 46 identisch ist. Auf diese Weise kann eine dezente und doch gut lesbare zweite Information geliefert werden. Insbesondere durch das Einschließen in eine farbige Zone 73 wird die zweite Information des zweiten Kennzeichens 74 besser wahrnehmbar, als wenn eine herkömmliche Ausgestaltung des zweiten Kennzeichens in Form einer herkömmlichen Bedruckung ohne den umschließenden Bereich des ersten Kennzeichens vorgesehen wäre. Durch den sich visuell vom zweiten Bereich 22 des Materials unterscheidbaren abhebenden ersten Bereich 21 des ersten Kennzeichens 72 wird ein Kontrast geschaffen, der die vergleichsweise kleinere schlechter erkennbare Information des zweiten Kennzeichens bezüglich der Wahrnehmbarkeit verbessert.

Das Kennzeichnungsmittel 70, vorzugweise in der Ausführung eines Einzelelements, weist in Querrichtung eine maximale Quererstreckung E1 und in Längsrichtung eine maximale Längserstreckung F1 auf. Dabei ist insbesondere vorgesehen, dass die maximale Querstreckung E1 des Kennzeichnungsmittels in Querrichtung der Inkontinenzvorlage größer ist als eine maximale Längserstreckung F1 des Kennzeichnungsmittels in Längsrichtung der Inkontinenzvorlage. Bei der Handhabung einer an sich ohnehin länglich erscheinenden Inkontinenzvorlage 2 wirkt ein Kennzeichnungsmittel 70 mit einer in Querrichtung der Inkontinenzvorlage ausgerichteten vergleichsweise größeren maximalen Querstreckung E1 als dessen Längserstreckung F1 in Längsrichtung der Inkontinenzvorlage prägnanter und ihm kommt damit eine größere Wahrnehmungskraft zu.

Der Wahrnehmung des Kennzeichnungsmittels innerhalb des hinteren Produktendabschnitts ist es zuträglich, wenn bei Betrachtung der Inkontinenzvorlage auf der Innenseite oder bei Betrachtung der Inkontinenzvorlage auf der Außenseite ein Unterschied an Ausgestaltung innerhalb des hinteren und vorderen Produktendabschnitts vorliegt, indem der vordere Produktendabschnitt entweder frei ist von farbiger Markierung oder im Falle einer farbigen Markierung dann eine für den Anwender unterscheidbare weniger prägnante Ausgestaltung der farbigen Markierungen im vorderen Produktendabschnitt gegeben ist im Vergleich zur farbigen Zone des Kennzeichnungsmittels im hinteren Produktendabschnitt.

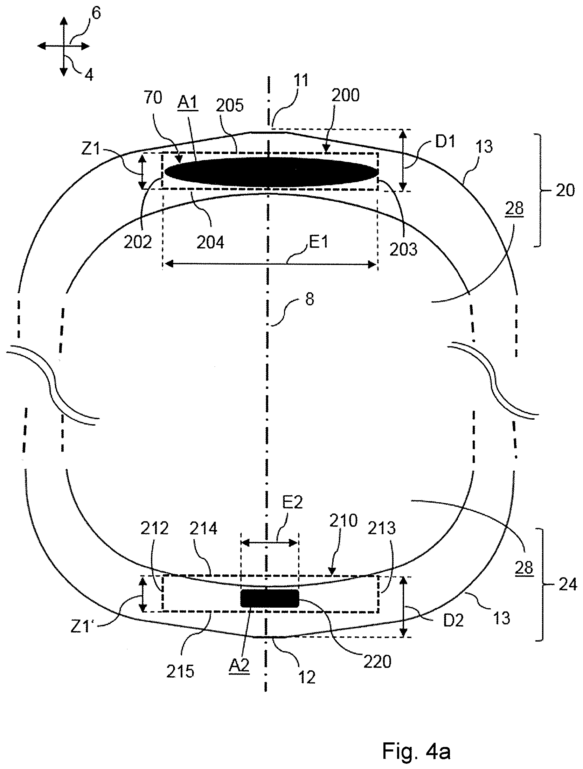

Die Figur 4a zeigt dazu eine vergleichende Betrachtung des im hinteren Produktendabschnitt 20 das Kennzeichnungsmittel 70 aufweisenden Bereichs mit einem entsprechenden in Längsrichtung 6 im vorderen Produktendabschnitt 24 verorteten Bereich.

Dabei wird der Vergleich der Bereiche innerhalb jeweils einer Oberseite, d.h. auf der Innenseite oder auf der Außenseite durchgeführt. Figur 4a zeigt die Aufsicht auf die Innenseite 28 im jeweiligen Ausschnitt des hinteren Produktendabschnitts 20 und des vorderen Produktendabschnitts 24.

Hierzu wird das Kennzeichnungsmittel 70, das eine Fläche A1 mit einer maximalen Quererstreckung E1 überfängt, von einem ersten imaginären Rechteck 200 gerade einbeschrieben ist, wobei das erste imaginäre Rechteck 200 erste und zweite Längsseiten 202, 203 parallel zu der Längsrichtung 4 und eine erste innere Querseite 204 und eine äußere Querseite 205 parallel zur Querrichtung 6 aufweist. Die erste und zweite Längsseite 204, 205 weisen die Länge Z1 auf und die erste innere Querseite 204 einen ersten Abstand D1 von einem hinteren Produktende 11. Als hinteres Produktende 11 wird dabei der in Längsrichtung 6 am weitest außen gelegene Punkt der Produktkonturlinie 13 verstanden, der in diesem Fall längsmittig auf der Längsmittelachse 8 liegt.

In dem vorderen Produktendabschnitt 24 wird auf derselben Innenseite 28 ein zweites imaginäres Rechteck 210 verortet, wobei das zweite imaginäre Rechteck 210 im Vergleich zum ersten imaginären Rechteck 200 parallel zur der Längsmittelachse 8 verschoben ist. Dabei weist das zweite imaginäre Rechteck 210 eine dritte und eine vierte Längsseite 212, 213 parallel zu der Längsrichtung 4 auf, und eine zweite innere Querseite 214 und zweite äußere Querseite 215 parallel zu der Querrichtung 6. Die dritte und vierte Längsseite 212, 213 weisen eine Länge Z1' auf, wobei die Länge Z1' der Länge Z1 des ersten imaginären Rechtecks 200 entspricht (Z1'=Z1). Die zweite innere Querseite 214 weist einen Abstand D2 von einem vorderen Produktende 12 auf. Das vordere Produktende 12 ist der in Längsrichtung 6 am weitest außen gelegene Punkt auf der Produktkonturlinie 13.

Für den Abstand D2 ist dabei eine Varianz derart vorgegeben, so dass gilt:
a) $D 2 \leq 1 , 5 x D 1$ und
b) die zweite äußere Querseite 215 des zweiten imaginären Rechtecks 210 darf sich nicht über das vordere Produktende 12 hinauserstrecken.

In Figur 4a ist das zweite imaginäre Rechteck 210 innerhalb des vorderen Produktendabschnitts 24 mit einem Abstand D2 verortet, wobei D2 dem Abstand D1 entspricht (D2 = D1).

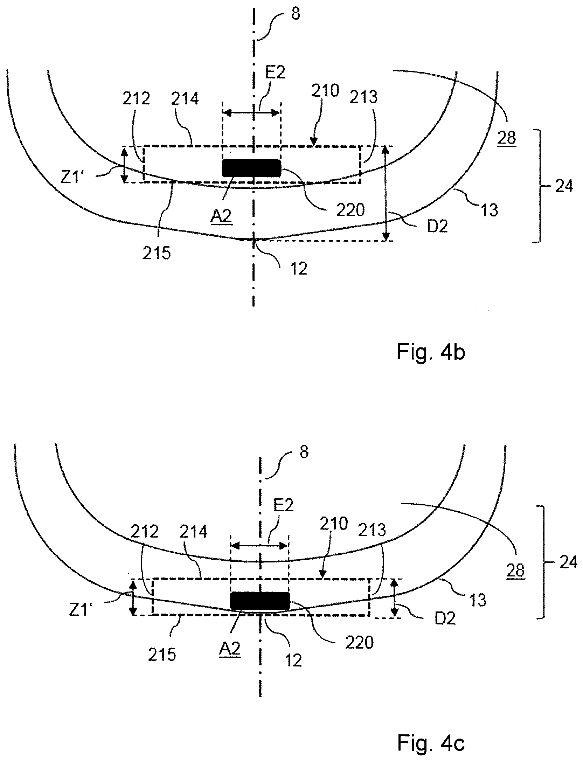

In den Figuren 4b und 4c sind die vorangehend genannten Varianzen des Abstandes D2, also die Grenzen innerhalb derer das zweite imaginäre Rechteck hinsichtlich des Abstandes D2 verortbar ist, schematisch innerhalb des vorderen Produktendabschnitts 24 dargestellt. So zeigt Figur 4b die Verortung des zweiten imaginären Rechtecks 210, bei der der Abstand D2 das eineinhalbfache des Abstandes D1 (D2 = 1,5 x D1) beträgt. Figur 4c zeigt die Anordnung des zweiten imaginären Rechtecks 210 derart, dass sich die zweite äußere Querkante 215 sich nicht über das vordere Produktende 12 hinauserstreckt, sondern D2 der Kantenlänge Z1 bzw. Z1' entspricht.

Für jede der Positionen, in die das zweite imaginäre Rechteck 210 verortet ist, ist vorzugsweise innerhalb des zweiten imaginären Rechtecks 210 keine visuelle erkennbare Markierung vorhanden.

Im Falle, dass eine visuell erkennbare Markierung 220 vorhanden ist, wie in den Figuren 4a-4c dargestellt, so ist die visuelle erkennbare Markierung 220 in ihrer Fläche A2 und/oder in ihrer maximalen Querstreckung E2 begrenzt. Die visuelle Markierung 220 weist eine Fläche A2 von höchstens 25% der Fläche A1 des Kennzeichnungsmittels 70 auf und/oder weist eine maximale Querstreckung E2 von höchstens 40% der maximalen Quererstreckung E1 des Kennzeichnungsmittels 70 auf.

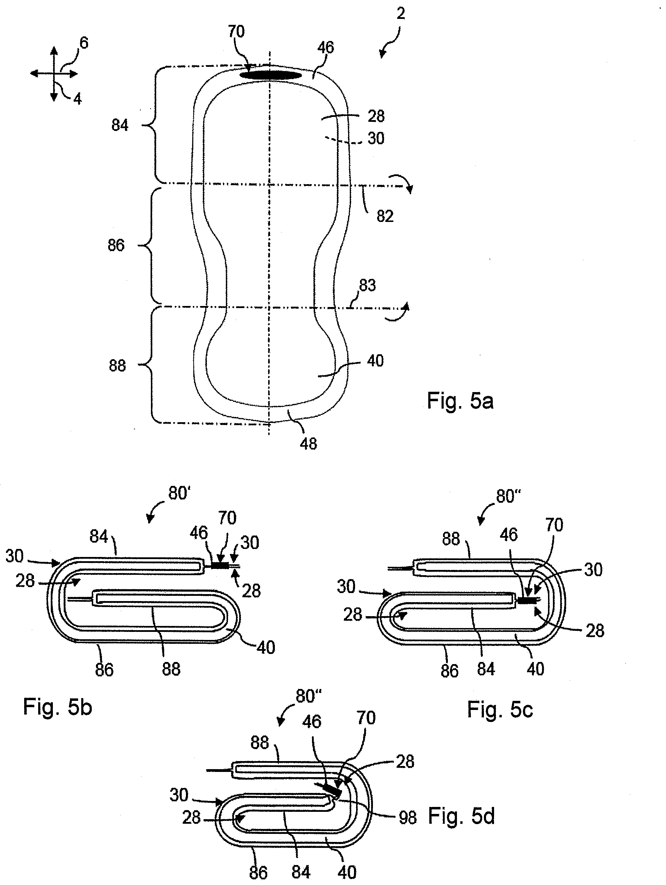

Für den Vertrieb der Inkontinenzvorlage 2 ist diese zumeist herstellerseitig in eine gefaltete Konfiguration verbracht. Figur 5a zeigt die Inkontinenzvorlage 2 von der Innenseite 28 vor der Überführung in eine gefaltete Konfiguration mit einem in Längsrichtung vorgesehenen hinteren Faltungsabschnitt 84, einem mittleren Faltungsabschnitt 86 und einem vorderen Faltungsabschnitt 88. Das Kennzeichnungsmittel 70 im hinteren Produktendabschnitt ist innerhalb des hinteren Faltungsabschnitts 84 angeordnet.

Bei Faltung um die in Querrichtung 6 verlaufenden Faltachsen 82, 83 kann dabei zuerst der hintere Faltungsabschnitt 84 um die Querfaltachse 82 einwärts auf die Innenseite 28 gefaltet werden oder zuerst der vordere Faltungsabschnitt 88 um die Querfaltachse 83 einwärts auf die Innenseite 28. Dem folgend wird der entsprechend andere vordere Faltungsabschnitt 88 bzw. hintere Faltungsabschnitt 84 um die jeweils andere Querfaltachse auf die Zwischenkonfiguration aufgefaltet. Daraus ergeben sich gefaltete Konfigurationen 80', 80", wie in Figur 5b und Figur 5b in einer Schnittansicht schematisch gezeigt ist, wobei der vordere Faltungsabschnitt 88 in der gefalteten Konfiguration 80' zwischen dem hinteren Faltungsabschnitt 84 und dem mittleren Faltungsabschnitt 86 angeordnet ist (Figur 5b) oder wobei der hintere Faltungsabschnitt 84 in der gefalteten Konfiguration 80" zwischen dem vorderen Faltungsabschnitt 88 und dem mittleren Faltungsabschnitt 86 angeordnet ist (Figur 5c). Das Kennzeichnungsmittel 70 kann zumindest bereichsweise sowohl von der Außenseite 30 als auch von der Innenseite 28 visuell erkannt werden, so dass eine Orientierung und eine Übermittlung der Informationen des Kennzeichnungsmittels 70 nach wie vor auch im gefalteten Zustand möglich ist. Sogar bei optional weiteren Faltungen, wie in der gefalteten Konfiguration 80" beispielsweise mit einer Faltung 89 innerhalb des hinteren Produktendabschnitt im Übergang zwischen einem den Saugkörper 80 aufweisenden Bereich zum flexibleren hinteren Quernahtabschnitt 46 (Figur 5d) , wird der Anwender nicht der Wahrnehmung der Information des Kennzeichnungsmittels 70 gestört, da das Kennzeichnungsmittel zumindest bereichsweise sowohl auf der Innenseite 28 als auch auf der Außenseite 30 visuell wahrnehmbar ist.

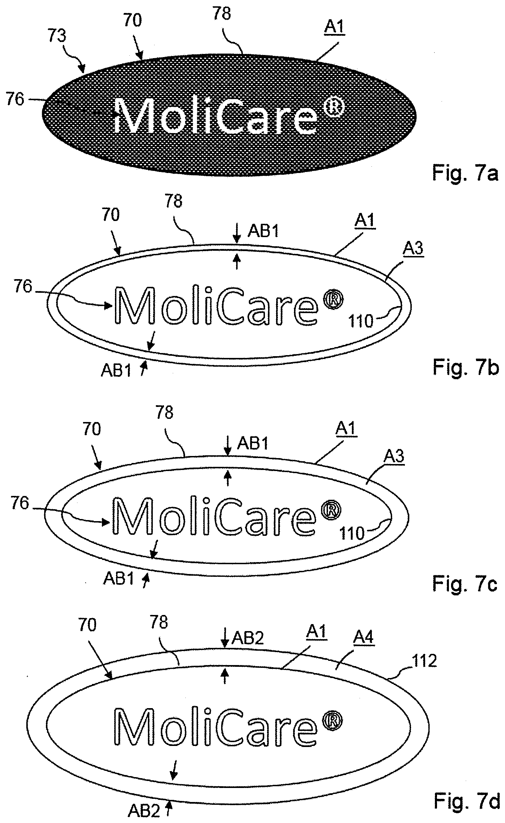

Für die Attraktion des Kennzeichnungsmittels ist eine gewisse flächenhafte Darstellung des ersten Kennzeichens wesentlich, insbesondere betrachtet ausgehend von einer Randumfangslinie 78 des Kennzeichnungsmittels. Ebenso vorteilhaft wird die Attraktion des Kennzeichnungsmittels bzw. damit auch des ersten Kennzeichens durch eine weitgehend solitäre Darstellung erreicht, also in einem äußeren Abstand von der Randumfangslinie 78 weitgehend frei von farbigen Zonen, insbesondere frei von weiteren farbigen Beschichtungen. Dies wird nachfolgend anhand Figuren 7a bis 7d erläutert.

Figuren 7a - 7d zeigen das Kennzeichnungsmittel 70, wobei in Figur 7a zu illustrativen Zwecken auf die Darstellung unten näher beschriebener imaginärer Abstandslinien verzichtet wurde und in den Figuren 7b - 7d zu illustrativen Zwecken die Darstellung der farbigen Zone 73 weggelassen wurde.

Die Fläche des Kennzeichnungsmittels 70, mithin die durch die Randumfangslinie 78 des ersten Kennzeichnungsmittels 70 einbeschriebene Fläche ist mit A1 bezeichnet. A3 (Figuren 7b und 7 c) bezeichnet die Fläche, welche von der Randumfangslinie 78 und einer ersten imaginären Abstandslinie 110 begrenzt wird, wobei die imaginäre Abstandslinie 110 innerhalb der Fläche A1 und in einem konstanten ersten Abstand AB1 von 2 mm nach innen beabstandet von der Randumfangslinie 78 verläuft. Die Größe der Fläche A3, gemessen in mm², beträgt im Falle der Figur 7b 20% der Größe der Fläche A1. In Figur 7c ist die imaginäre Anstandslinie 110 in größerem ersten Abstand AB1 von 3 mm von der Randumfangslinie 78 des ersten Kennzeichnungsmittels 70 skizziert. Die Größe der Fläche A3 beträgt hier 30% der Größe der Fläche A1.

Die Fläche A3 sollte im Wesentlichen durch das erste Kennzeichen gebildet und ausgefüllt sein; der Flächenanteil des zweiten Kennzeichens (also der Aussparungen 76), an der Größe der Fläche A3 beträgt vorteilhaft höchstens 10%, weiter insbesondere höchstens 7%, weiter insbesondere höchstens 5%.

In Figur 7d bezeichnet A4 die Fläche, welche von der Randumfangslinie 78 des Kennzeichnungsmittels 70 und einer zweiten imaginären Abstandslinie 112 begrenzt wird, wobei die zweite imaginäre Abstandslinie 112 außerhalb der Fläche A1 und in einem konstanten zweiten Abstand AB2 von 5 mm beabstandet von der Randumfangslinie 78 verläuft. Die Größe der Fläche A4, gemessen in mm², beträgt hier in etwa 50% der Größe der Fläche A1. Die Fläche A4 ist im Wesentlichen, insbesondere zu mindestens 90%, insbesondere zu 100% frei von weiteren farbigen Zonen, insbesondere frei von farbigen Beschichtungen und/oder Bedruckungen.

Figuren 8a - 8c zeigen nun in einzelnen Schritten eine Bestimmung des Anteils des zweiten Kennzeichens und dessen Verteilung an der Fläche des Kennzeichnungsmittels 70. Hierzu wird das Kennzeichnungsmittel 70 (Figur 8a) durch ein Rechteck 100 tangierend umschlossen (Fig. 8b), das bezüglich seiner Kanten in Längs- und Querrichtung 4 bzw. 6 der Inkontinenzvorlage 2 verläuft. Mit dem Rechteck 100 werden die jeweils äußersten Punkte des Kennzeichnungsmittels in dessen Quer- und Längsstreckung gerade einbeschrieben. Es erfolgt dann eine Teilung in gleich große Unterrechtecke des Rechtecks 100, die hier mit 101 bezeichnet sind, wobei sowohl in Längs- als auch in Querrichtung 4, 6 jeweils zehn Unterrechtecke 101 vorgesehen sind. Es wird anschließend die Anzahl N1 an Unterrechtecken 101a ermittelt, welche vollständig oder zumindest bereichsweise innerhalb der Randumfangslinie 78 des Kennzeichnungsmittels 70 lokalisiert sind. Es wird dann weiter innerhalb dieser die Anzahl N1 der Unterrechtecke 101a die Anzahl N2 derjenigen Unterrechtecke 101b bestimmt (in Figur 8c), welche bereichsweise sowohl das erste Kennzeichen als auch das zweite Kennzeichen aufweisen. Im vorliegenden Fall sind N1 = 88 Unterrechtecke 101a vorhanden, davon sind N2 = 27 Unterrechtecke 101b sowohl mit dem ersten Kennzeichen als auch mit dem zweiten Kennzeichen versehen. Diese 27 Unterrechtecke 101b sind in Figur 8c zur Veranschaulichung dunkel unterlegt. Somit liegt ein Verteilungsgrad von N2/N1*100% von 31 % vor.

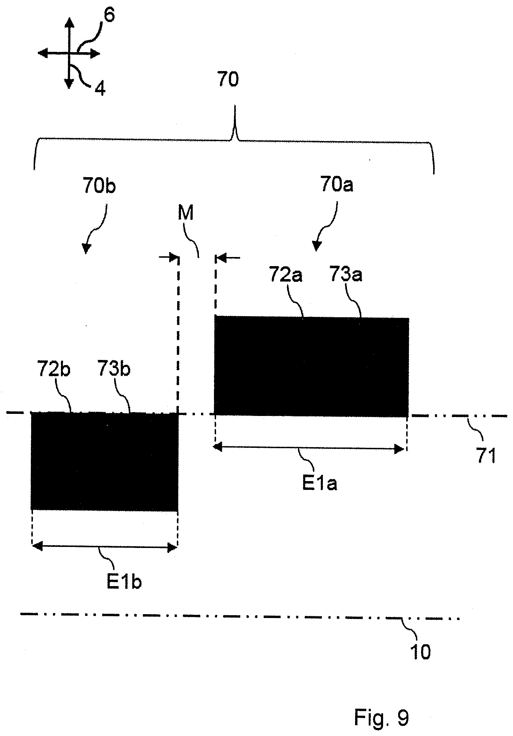

Das Kennzeichnungsmittel 70 kann alternativ zu dem in den vorangehenden Figuren Gezeigtem, wobei das Kennzeichnungsmittel als ein Einzelelement ausgebildet, auch eine Anordnung von zwei Sub-Kennzeichnungsmittel 70a, 70b, weiter insbesondere eine Vielzahl an Sub-Kennzeichnungsmittel aufweisen. Die funktionale Verbundenheit von mehreren Sub-Kennzeichnungsmittel 70a, 70b zu einem Kennzeichnungsmittel 70 ist dadurch gegeben, wie in Figur 9 schematisch dargestellt ist,
- dass die von zwei Sub-Kennzeichnungsmittel 70a, 70b benachbarten farbigen Zonen 73a, 73b der jeweiligen ersten Kennzeichen 72a, 72b auf einer parallel zur Quermittelachse 10 in Querrichtung 6 der Inkontinenzvorlage verlaufenden Linie 71 angeordnet sind und
- dass bei einem ersten Sub-Kennzeichnungsmittel 70a mit einer maximalen Quererstreckung E1a und bei einem benachbarten zweiten Sub-Kennzeichnungsmittel 70b mit einer maximalen Querstreckung E1b, der Abstand M zwischen den beiden Sub-Kennzeichnungsmittel 70 a, 70b als die kürzeste Distanz in Querrichtung 6 zwischen den benachbarten farbigen Zonen höchstens 25 %, insbesondere höchstens 20%, insbesondere höchstens 15%, insbesondere höchstens 10% der ersten oder zweiten maximalen Quererstreckung E1a oder E1b beträgt, dabei im Falle von unterscheidbaren ersten und zweiten Querstreckung E1a und E1b auf die kleinere maximale Quererstreckung von beiden maximalen Querstreckung E1a und E1b bezogen ist.

"Auf einer zur Quermittelachse parallelen Linie angeordnet" versteht sich als Positionierung der farbigen Zone, bei der diese Linie die farbige Zone in ihrer flächenhaften Erstreckung zumindest tangiert, vorzugsweise durchkreuzt.

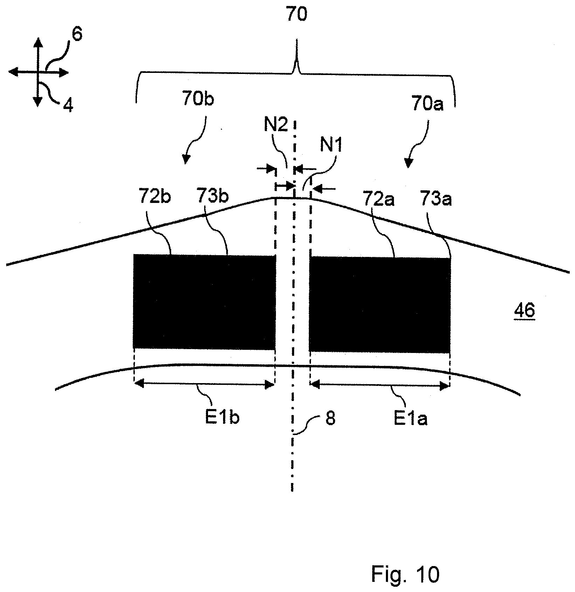

Insbesondere kann ein an der Längsmittelachse 8 angeordnetes Kennzeichnungsmittel 70 eine Anordnung aus zwei Sub-Kennzeichnungsmittel 70a, 70b mit je einem ersten Kennzeichen 72a, 72b aufweisen, wie schematisch in Figur 10 dargestellt ist.

Die zwei Sub-Kennzeichnungsmittel 70a 70 b gleichen sich dabei insbesondere hinsichtlich eines Abstandes N1, N2 des einzelnen Sub-Kennzeichnungsmittels zur Längsmittelachse und/oder insbesondere hinsichtlich einer maximale Querstreckung E1a, E1b des jeweiligen Sub-Kennzeichnungsmittels und /oder insbesondere hinsichtlich einer vom jeweiligen Sub-Kennzeichnungsmittel eingeschriebenen Fläche und/oder insbesondere hinsichtlich einer vom ersten Kennzeichen eingeschriebenen Fläche und Kontur, also Form der farbigen Zone 73a, 73 b. Insbesondere sind die zwei Sub-Kennzeichnungsmittel im Wesentlichen zueinander symmetrisch an der Längsmittelachse angeordnet.

Der jeweilige Abstand N1, N2 des einzelnen Sub-Kennzeichnungsmittels zur Längsmittelachse beträgt höchstens 20%, insbesondere höchstens 15%, insbesondere höchstens 10% der maximalen Querstreckung des Sub-Kennzeichnungsmittels.

Das erste Kennzeichen eines Kennzeichnungsmittels, gebildet durch eine flächenhaft erstreckte farbige Zone in einem ersten Bereich des Rückenbereichs der Inkontinenzvorlage, unterscheidet sich bei Aufsicht auf die Produktaußenseite und bei Aufsicht auf die Produktinnenseite von dem das erste Kennzeichen direkt umgebenden zweiten Bereich vorteilhafterweise in den nach CIEL*a*b* zueinander ermittelten Farbabständen, dargestellt als ΔE*. Vorzugsweise beträgt der Farbstand 15 - 55. Damit kommt dem Kennzeichnungsmittel eine gute Signalwirkung zu.

In der bevorzugten Ausführungsform des Kennzeichnungsmittels 70 mit einem innerhalb des ersten Kennzeichens durch Aussparungen erhaltenen zweiten Kennzeichens schaffen nach CIEL*a*b* ermittelte Farbabstände von vorzugsweise 15 - 55 einen Kontrast zwischen dem ersten und zweiten Kennzeichen, was die Wahrnehmbarkeit des zweiten Kennzeichens verbessert.

Beispielhaft ist das Kennzeichnungsmittel 70 in einem hinteren Quernahtabschnitt 46 mit einem Lagenaufbau, wie schematisch in Figur 3c gezeigt, umfassend ein Deckblatt-Material 32 aus Vliesmaterial und ein textiles Backsheet-Material 34 mit einer inneren Folienlage 35 und einer äußeren Vlieslage 36 auf der der Außenseite 30 zugewandten Oberseite der inneren Folienlage angebracht. Dabei ist die flächenhafte erstreckte farbige Zone 73 des ersten Kennzeichens 72 bei einer ersten Inkontinenzvorlage mit einer blau farbenen Druckfarbe eingebracht, bei einer zweiten Inkontinenzvorlage mit einer grün farbenen Druckfarbe.

Das Deckblatt-Material aus einem PP-Spinnvlies weist ein Flächengewicht von 12-14 g/m² und eine Opazität von 22- 26% auf, das textile Backsheet-Material mit einer PE-Folie und einem PP-Spinnvlies weist ein Flächengewicht von 18 g/m² und eine Opazität von 50-60% auf.

Das erste und zweite Kennzeichen eines Kennzeichnungsmittels und der das Kennzeichnungsmittel direkt umgebende zweite Bereich unterscheiden sich bei Aufsicht auf das Produkt von der Außenseite und der Innenseite in deren Farben, gemessen nach CIEL*a*b* und den zueinander ermittelten Farbabständen, dargestellt als ein jeweiliges ΔE*. In Tabelle 1 und 2 sind dazu die Ergebnisse dargestellt.

**Tabelle 1: Beispiele für Ausführungsformen mit erstem und zweitem Kennzeichen und deren Farbbestimmung nach CIEL*a*b***

| Vorlage 1 | Kennzeichen 1 Blau P540 | | | Kennzeichen 2 / direkt umgebender Bereich | | |
|---|---|---|---|---|---|---|
| | *L | *a | *b | *L | *a | *b |
| Produktaußenseite | 49,61 | 2,13 | -11,42 | 92,96 | -0,68 | 2,05 |
| Produktinnenseite | 62,96 | -1,27 | -9,35 | 92,46 | -0,71 | 2,32 |
| | | | | | | |

| Vorlage 2 | Kennzeichen 1 Grün P3282 | | | Kennzeichen 2 / direkt umgebender Bereich | | |
|---|---|---|---|---|---|---|
| | *L | *a | *b | *L | *a | *b |
| Produktaußenseite | 60,25 | -22,21 | -2,14 | 92,96 | -0,68 | 2,05 |
| Produktinnenseite | 67,5 | -12,85 | -4,51 | 92,46 | -0,71 | 2,32 |

**Tabelle 2: Farbabstände von erstem Kennzeichen 1 zu zweitem Kennzeichen 2 bzw. direkt umgebenden Bereich, dargestellt als Delta E und daraus ermittelte Unterschiede**

| | Farbabstand | Vorlage 1 (blaues Kennzeichen 1) | Vorlage 2 (grünes Kennzeichen 1) |
|---|---|---|---|
| Produktaußenseite | Delta E (Δ E*) | 45,5 | 39,4 |
| Produktinnenseite | Delta E (Δ E*) | 31,7 | 28,6 |
| | | | |
| Differenz Delta E (Δ E*) Produktaußenseite - Produktinnenseite | | 13,76 | 10,80 |

## Patentansprüche

1. Wegwerfbare absorbierende Inkontinenzvorlage (2) mit einer Längsrichtung (4) und einer Querrichtung (6), einer Längsmittelachse (8) und einer Quermittelachse (10) und mit einer Produktlänge L1 und mit einer Produktbreite B1, mit einem entlang der Längsrichtung angeordneten hinterem Produktabschnitt (14), einem mittleren Produktabschnitt (15) und einem vorderen Produktabschnitt (16), wobei jeder Produktabschnitt jeweils ein Drittel der Produktlänge L1 erfasst, mit einem hinteren Produktendabschnitt (20) und einem vorderen Produktendabschnitt (24), welche in Längsrichtung jeweils ein Zehntel (1/10) der Produktlänge L1 erfassen, mit einer Innenseite (28) und einer Außenseite (30),mit einem Deckblatt-Material (32), einem Backsheet-Material (34) und einem dazwischen angeordneten Saugkörper (40), wobei im hinteren Produktendabschnitt (20) ein Kennzeichnungsmittel (70) vorgesehen ist, das an der Längsmittelachse (8) angeordnet ist, wobei innerhalb des hinteren Produktendabschnitts (20) ein hinterer Quernahtabschnitt (46) vorgesehen ist, wobei der hintere Quernahtabschnitt (46) einen Überhang (38) umfasst oder daraus gebildet ist, wobei der Überhang (38) einen Bereich (39) umfasst oder daraus gebildet ist, in dem das Deckblatt-Material (32) und/oder das Backsheet-Material (34) sich außerhalb einer den Saugkörper (40) begrenzenden Konturlinie (42) erstreckt, wobei das Kennzeichnungsmittel (70) innerhalb des hinteren Produktendabschnitts (20) zumindest bereichsweise in dem hinteren Quernahtabschnitt (46) angeordnet ist, wobei das Kennzeichnungsmittel (70) in einem ersten Bereich (21) des hinteren Produktendabschnittes (20) ein erstes Kennzeichen (72) aufweist, das durch eine flächenhaft erstreckte farbige Zone (73) gebildet ist und das eine erste Information bereitstellt, wobei die farbige Zone (73) visuell unterscheidbar ist von einem das erste Kennzeichen (72) umgebenden zweiten Bereich (22) des hinteren Produktendabschnitts (20), und wobei das Kennzeichnungsmittel (70) zumindest bereichsweise sowohl auf der Innenseite (28) als auch auf der Außenseite (30) visuell erkennbar ist,
**dadurch gekennzeichnet, dass** auf der Innenseite (28) oder dass auf der Außenseite (30) das Kennzeichnungsmittel (70) eine Fläche A1 mit einer maximalen Querstreckung E1 aufweist,
- und dass das Kennzeichnungsmittel von einem ersten imaginären Rechteck (200) gerade einbeschrieben ist, wobei das erste imaginäre Rechteck (200) erste und zweite Längsseiten (202, 203) parallel zu der Längsrichtung (4) aufweist und eine erste innere Querseite (204) und eine äußere Querseite (205) parallel zur Querrichtung (6) aufweist und wobei die erste innere Querseite (204) einen ersten Abstand D1 von einem hinteren Produktende (11) aufweist und wobei die erste und zweite Längsseite (204, 205) eine Länge Z1 aufweisen,
- und dass ein zweites imaginäres Rechteck (210) auf derselben Innenseite (28) oder Außenseite (30) in dem vorderen Produktendabschnitt (24) in einer Position verortbar ist, wobei das zweite imaginäre Rechteck (210) eine dritte und eine vierte Längsseite (212, 213) parallel zu der Längsrichtung (4) aufweist, und eine zweite innere Querseite (214) und zweite äußere Querseite (215) parallel zu der Querrichtung (6) aufweist, und wobei die dritte und vierte Längsseite (212, 213) eine Länge Z1' aufweisen, die der Länge Z1 entspricht (Z1'=Z1) und wobei die zweite innere Querseite (214) einen Abstand D2 von einem vorderen Produktende (12) aufweist, und dass für jede der Positionen, in die das zweite imaginäre Rechteck (210) verortet wird, gilt:
a) entweder innerhalb des zweiten imaginären Rechtecks (210) keine visuell erkennbare farbige Markierung (220) vorhanden ist, oder
b) im Falle einer visuell erkennbaren farbigen Markierung (220) innerhalb des zweiten imaginären Rechtecks (210) die farbige Markierung (220) eine Fläche A2 mit einem Flächenanteil von höchstens 25%, insbesondere höchstens 20%, weiter insbesondere höchstens 15%, weitere insbesondere höchstens 10 % der Fläche A1 aufweist
und/oder
c) im Falle einer visuell erkennbaren farbigen Markierung (220) innerhalb des zweiten imaginären Rechtecks (210), die farbige Markierung (220) eine maximale Querstreckung E2 aufweist, wobei die maximale Querstreckung E2 kleiner ist als die maximale Quererstreckung E1 des Kennzeichnungsmittels (70), wobei insbesondere die maximale Quererstreckung E2 höchstens 40 %, insbesondere höchstens 30%, weiter insbesondere höchstens 20%, weitere insbesondere höchstens 10 % der maximalen Querstreckung E1 beträgt,
- wobei die Positionen, in die das zweite imaginäre Rechteck (210) verortet wird, dadurch begrenzt sind, dass das zweite imaginäre Rechteck (210) im Vergleich zum ersten imaginären Rechteck (200) parallel zur der Längsmittelachse (8) verschoben ist und für den Abstand D2 gilt:
a) $D 2 \leq 1 , 5 x D 1$ und
b) die zweite äußere Querseite (215) des zweiten imaginären Rechtecks (210) sich nicht über das vordere Produktende (12) hinauserstreckt.

2. Inkontinenzvorlage nach einem der vorherigen Ansprüche, **dadurch gekennzeichnet, dass** das Kennzeichnungsmittel (70) ein Einzelelement ist.

3. Inkontinenzvorlage nach einem der vorherigen Ansprüche, **dadurch gekennzeichnet, dass** das Kennzeichnungsmittel (70) ein zweites Kennzeichen (74) umfasst, das eine zweite Information bereitstellt, das dadurch gebildet ist, dass in dem ersten Kennzeichen (72) Aussparungen (76) der farbigen Zone (73) vorgesehen sind, wobei insbesondere das zweite Kennzeichen (74) alphanummerische Zeichen umfasst.

4. Inkontinenzvorlage nach einem der vorherigen Ansprüche, **dadurch gekennzeichnet, dass** das Kennzeichnungsmittel (70) auf dem Backsheet-Material (34), dabei auf einer der Innenseite (28) zugewandten Oberseite oder einer der Außenseite (30) zugewandten Oberseite des Backsheet-Materials (34) angebracht ist, oder insbesondere bei einem Backsheet-Material (34) umfassend eine innere Folienlage (35) und eine äußere Vlieslage (36) das Kennzeichnungsmittel (70) dazwischen und dabei insbesondere auf der der Außenseite (30) zugewandten Oberseite der Folienlage (35) angebracht ist oder dass das Kennzeichnungsmittel (70) auf dem Deckblatt-Material (32), dabei insbesondere auf einer der Außenseite (30) zugewandten Oberseite des Deckblatt-Materials (32) angebracht ist.

5. Inkontinenzvorlage nach einem der vorherigen Ansprüche, **dadurch gekennzeichnet, dass** die farbige Zone (73) des Kennzeichnungsmittels (70) eine Bedruckung, insbesondere eine farbige Bedruckung ist.

6. Inkontinenzvorlage nach einem der vorherigen Ansprüche, **dadurch gekennzeichnet, dass** das Kennzeichnungsmittels (70) bzw. die farbige Zone (73) des ersten Kennzeichens (74) des Kennzeichnungsmittels eine maximale Quererstreckung E1 von höchstens 1/2, insbesondere höchstens 1/3, insbesondere höchstens ¼ einer maximalen Quererstreckung B2 des hinteren Produktabschnitts (14), und insbesondere mindestens 1/6 einer maximalen Querstreckung B2 des hinteren Produktabschnitts (14) aufweist.

7. Inkontinenzvorlage nach einem der vorherigen Ansprüche, **dadurch gekennzeichnet, dass** eine maximale Querstreckung E1 des Kennzeichnungsmittels (70) in Querrichtung (6) der Inkontinenzvorlage größer ist als eine maximale Längserstreckung F1 des Kennzeichnungsmittels in Längsrichtung (4) der Inkontinenzvorlage.

8. Inkontinenzvorlage nach einem der vorherigen Ansprüche, **dadurch gekennzeichnet, dass** das Kennzeichnungsmittel (70) eine maximale Längserstreckung F1 aufweist und mit mindestens 50% der maximalen Längserstreckung F1, vorzugsweise mindestens 60%, weitere vorzugsweise mindestens 70%, weiter vorzugsweise mindestens 80%, weiter vorzugsweise mindestens 90% innerhalb eines hinteren Quernahtabschnitts (46) angeordnet ist, insbesondere bevorzugt ist das Kennzeichnungsmittel (70) vollumfänglich innerhalb eines hinteren Quernahtabschnitts (48) angeordnet.

9. Inkontinenzvorlage nach einem der vorherigen Ansprüche, **dadurch gekennzeichnet, dass** der hintere Produktabschnitt (14) eine maximale Querstreckung B2 und der vordere Produktabschnitt (16) eine maximale Querstreckung B3 aufweist, wobei ein Quotient von B2/B3
entweder a) einen Wert ≥ 1 und vorzugsweise einen Wert ≤ 1,50, vorzugsweise ≤ 1,45, vorzugsweise ≤ 1,40, vorzugsweise ≤ 1,35, vorzugsweise ≤ 1,30, vorzugsweise ≤ 1,25 aufweist
oder b) einen Wert ≤ 1 und vorzugsweise ≥ 0,75, vorzugsweise ≥ 0,80 aufweist.

10. Inkontinenzvorlage nach einem der vorherigen Ansprüche, **dadurch gekennzeichnet, dass** die Produktlänge L1 450 - 800 mm, vorzugsweise 500 - 800 mm, weiter vorzugsweise 500 - 750 mm beträgt und/oder dass die Produktbreite B1 200 - 450 mm, vorzugsweise 250 - 450 mm, weiter vorzugsweise 280 - 400 mm beträgt.

11. Inkontinenzvorlage nach einem der vorherigen Ansprüche 1-10, **dadurch gekennzeichnet, dass** der hintere Quernahtabschnitt (46) einen Flächenanteil von 3- 8%, bevorzugt 4 - 7 %, weiter bevorzugt 4- 6 % bezogen auf eine Produkt-Fläche der Inkontinenzvorlage aufweist und/oder dass der hintere Quernahtabschnitt (46) einen Flächenanteil von 45 - 65%, bevorzugt 48 -63 %, weiter bevorzugt 50 - 60% bezogen auf eine Fläche des hinteren Produktendabschnitt (20) aufweist, insbesondere dass der hintere Quernahtabschnitt (46) eine Fläche von 4.000 - 14.000 mm², vorzugsweise 5.000 - 12.000 mm², weiter vorzugsweise 5.000 -11.000 mm² aufweist.

12. Inkontinenzvorlage nach einem der vorherigen Ansprüche 1-11, **dadurch gekennzeichnet, dass** das Kennzeichnungsmittel (70) 15- 80 %, vorzugsweise 20 - 70 %, weiter vorzugsweise 25 - 60 %, weiter vorzugsweise 30 - 50 % einer Fläche des hinteren Quernahtabschnitts (46) überfängt und/oder dass das Kennzeichnungsmittel (70) 8 - 60 %, insbesondere 10 - 55 %, weiter insbesondere 10 - 50 %, weiter insbesondere 10 - 40 % einer Fläche des hinteren Produktendabschnitts (20) überfängt.

13. Inkontinenzvorlage nach einem der vorherigen Ansprüche 3 - 12, **dadurch gekennzeichnet, dass** die farbige Zone (73) ausgehend von einer Randumfangslinie (78) des Kennzeichnungsmittels (70) das zweite Kennzeichen (74) derart umschließt, dass die Größe einer Fläche A3, welche von der Randumfangslinie (78) des Kennzeichnungsmittels und einer davon innerhalb und in einem konstanten ersten Abstand AB1 verlaufenden imaginären inneren Abstandslinie (110) begrenzt wird, mindestens 10%, insbesondere mindestens 15%, insbesondere mindestens 20%, insbesondere mindestens 25%, weiter insbesondere mindestens 30% einer Größe der Fläche A1 beträgt, weiter vorzugsweise die Fläche A3 höchstens 50%, weiter insbesondere höchstens 45%, weiter insbesondere höchstens 40%, weiter insbesondere höchstens 35% einer Größe der Fläche A1 beträgt, wobei als die Fläche A1 die durch die Randumfangslinie (78) des Kennzeichnungsmittels (70) eingeschriebene Fläche definiert ist, wobei der Flächenanteil des zweiten Kennzeichens (74) an der Fläche A3 höchstens 10%, insbesondere höchstens 7%, weiter insbesondere höchstens 5%, insbesondere höchstens 2% beträgt und/oder **dadurch gekennzeichnet, dass** die Größe einer Fläche A4, welche von einer Randumfangslinie (78) des Kennzeichnungsmittels (70) und einer davon außerhalb und in einem konstanten zweiten Abstand AB2 verlaufenden imaginären Abstandslinie (112) begrenzt wird, mindestens 30%, insbesondere mindestens 40%, insbesondere mindestens 50%, insbesondere mindestens 60%, weiter insbesondere mindestens 70% der Größe einer Fläche A1 beträgt, wobei als die Fläche A1 die durch die Randumfangslinie (78) des Kennzeichnungsmittels (70) eingeschriebene Fläche definiert ist, wobei vorzugsweise mindestens 90%, insbesondere mindestens 93%, insbesondere mindestens 95% der Fläche A4 frei von weiteren farbigen Zonen, insbesondere als Beschichtungen und/oder Bedruckungen und/oder Änderungen der dem Deckblatt-Material und/oder Backsheet-Material zu eigenen optischen visuellen und/oder haptischen Beschaffenheit sind.

14. Inkontinenzvorlage nach einem der vorherigen Ansprüche 3 - 13, **dadurch gekennzeichnet, dass** das Kennzeichnungsmittel (70) eine durch eine Randumfangslinie (78) eingeschriebene Fläche A1 überfängt, und das zweite Kennzeichen (74) einen Flächenanteil von höchstens 50%, insbesondere höchstens 40%, weiter insbesondere höchstens 30%, weiter insbesondere höchstens 20%, und insbesondere wenigstens 3 %, insbesondere wenigstens 5 %, insbesondere wenigstens 8 % der vom Kennzeichnungsmittel überfangenen Fläche A1 einnimmt.

15. Inkontinenzvorlage nach einem der vorherigen Ansprüche 3 - 14, **dadurch gekennzeichnet, dass** das Kennzeichnungsmittel (70) mit einer Randumfangslinie (78) in seinen jeweils äußersten Punkten von einem imaginären Rechteck (100) tangierend eingeschrieben ist, wobei das Rechteck (100) bezüglich seiner Kanten in Längs- und Querrichtung des Inkontinenzartikels angeordnet ist und dabei sowohl in Längsrichtung als auch in Querrichtung in jeweils 10 gleich große Unterrechtecke (101) unterteilt ist, von einer Anzahl N1 an Unterrechtecken (101a), welche vollständig oder zumindest bereichsweise innerhalb der Randumfangslinie (78) lokalisiert sind und davon einer Anzahl N2 derjenigen Unterrechtecken (101b), die zumindest bereichsweise sowohl das erste Kennzeichen (72) als auch das zweite Kennzeichen (74) aufweisen, ein Verteilungsgrad von N2/N1*100% von mindestens 10 %, insbesondere mindestens 20 %, insbesondere mindestens 25%, insbesondere mindestens 30%, insbesondere mindestens 35%, weiter insbesondere von höchstens 60%, weiter insbesondere höchstens 55 %, weiter insbesondere höchstens 50%, weiter insbesondere höchstens 45% vorgesehen ist.

## Claims

1. Absorbent disposable incontinence pad (2) having a longitudinal direction (4) and a transverse direction (6), a longitudinal central axis (8) and a transverse central axis (10), and having a product length L1 and having a product width B1, having a rear product portion (14) arranged along the longitudinal direction, a central product portion (15) and a front product portion (16), wherein each product portion covers one third of the product length L1, having a rear product end portion (20) and a front product end portion (24), which each cover one tenth (1/10) of the product length L1 in the longitudinal direction, having an inner side (28) and an outer side (30), having a cover sheet material (32), a backsheet material (34) and an absorbent body (40) arranged inbetween, wherein an identification means (70) is provided in the rear product end portion (20), said identification means being arranged on the longitudinal central axis (8), wherein a rear transverse seam portion (46) is provided within the rear product end portion (20), wherein the rear transverse seam portion (46) comprises or is formed from an overhang (38), wherein the overhang (38) comprises or is formed from a region (39) in which the cover sheet material (32) and/or the backsheet material (34) extends/extend outside a contour line (42) delimiting the absorbent body (40), wherein the identification means (70) is arranged within the rear product end portion (20) at least regionally in the rear transverse seam portion (46), wherein the identification means (70) has, in a first region (21) of the rear product end portion (20), a first identifier (72) which is formed by a two-dimensionally extending coloured zone (73) and which provides first information, wherein the coloured zone (73) is visually distinguishable from a second region (22) of the rear product end portion (20) surrounding the first identifier (72), and wherein the identification means (70) is visually recognizable at least regionally both on the inner side (28) and on the outer side (30),
**characterized in that**, on the inner side (28) or **in that**, on the outer side (30), the identification means (70) has an area A1 with a maximum transverse extent E1,
- and **in that** the identification means is specifically inscribed by a first imaginary rectangle (200), wherein the first imaginary rectangle (200) has first and second longitudinal sides (202, 203) parallel to the longitudinal direction (4) and has a first inner transverse side (204) and an outer transverse side (205) parallel to the transverse direction (6), and wherein the first inner transverse side (204) is at a first spacing D1 from a rear product end (11), and wherein the first and second longitudinal sides (204, 205) have a length Z1,
- and **in that** a second imaginary rectangle (210) can be located in a position on the same inner side (28) or outer side (30) in the front product end portion (24), wherein the second imaginary rectangle (210) has a third and a fourth longitudinal side (212, 213) parallel to the longitudinal direction (4) and has a second inner transverse side (214) and a second outer transverse side (215) parallel to the transverse direction (6), and wherein the third and fourth longitudinal sides (212, 213) have a length Z1' which corresponds to the length Z1 (Z1'=Z1), and wherein the second inner transverse side (214) is at a spacing D2 from a front product end (12), and **in that** the following applies to each of the positions in which the second imaginary rectangle (210) is located:
a) there is either no visually recognizable coloured marking (220) within the second imaginary rectangle (210), or
b) if there is a visually recognizable coloured marking (220) within the second imaginary rectangle (210), the coloured marking (220) has an area A2 with a proportion of at most 25%, in particular at most 20%, more particularly at most 15%, more particularly at most 10%, of the area A1,
and/or
c) if there is a visually recognizable coloured marking (220) within the second imaginary rectangle (210), the coloured marking (220) has a maximum transverse extent E2, wherein the maximum transverse extent E2 is smaller than the maximum transverse extent E1 of the identification means (70), wherein in particular the maximum transverse extent E2 is at most 40%, in particular at most 30%, more particularly at most 20%, more particularly at most 10%, of the maximum transverse extent E1,
- wherein the positions in which the second imaginary rectangle (210) is located are delimited by the second imaginary rectangle (210) being displaced parallel to the longitudinal central axis (8) in comparison to the first imaginary rectangle (200), and the following applies with regard to the spacing D2:
a) D2 ≤ 1.5 x D1, and
b) the second outer transverse side (215) of the second imaginary rectangle (210) does not extend beyond the front product end (12).

2. Incontinence pad according to one of the preceding claims, **characterized in that** the identification means (70) is an individual element.

3. Incontinence pad according to one of the preceding claims, **characterized in that** the identification means (70) comprises a second identifier (74) which provides second information and which is formed by clearances (76) of the coloured zone (73) being provided in the first identifier (72), wherein in particular the second identifier (74) comprises alphanumeric characters.

4. Incontinence pad according to one of the preceding claims, **characterized in that** the identification means (70) is fixed on the backsheet material (34), in this case is fixed on a top side of the backsheet material (34) facing the inner side (28) or on a top side thereof facing the outer side (30), or, in particular in the case of a backsheet material (34) comprising an inner film ply (35) and an outer nonwoven ply (36), the identification means (70) is fixed inbetween and in particular on the top side of the film ply (35) facing the outer side (30), or **in that** the identification means (70) is fixed on the cover sheet material (32), in particular on a top side of the cover sheet material (32) facing the outer side (30).

5. Incontinence pad according to one of the preceding claims, **characterized in that** the coloured zone (73) of the identification means (70) is a print, in particular a coloured print.

6. Incontinence pad according to one of the preceding claims, **characterized in that** the identification means (70) or the coloured zone (73) of the first identifier (74) of the identification means has a maximum transverse extent E1 of at most 1/2, in particular at most 1/3, in particular at most ¼ of a maximum transverse extent B2 of the rear product portion (14), and in particular at least 1/6 of a maximum transverse extent B2 of the rear product portion (14).

7. Incontinence pad according to one of the preceding claims, **characterized in that** a maximum transverse extent E1 of the identification means (70) in the transverse direction (6) of the incontinence pad is greater than a maximum longitudinal extent F1 of the identification means in the longitudinal direction (4) of the incontinence pad.

8. Incontinence pad according to one of the preceding claims, **characterized in that** the identification means (70) has a maximum longitudinal extent F1 and is arranged with at least 50% of the maximum longitudinal extent F1, preferably at least 60%, more preferably at least 70%, more preferably at least 80%, more preferably at least 90%, within a rear transverse seam portion (46); particularly preferably, the identification means (70) is arranged entirely within a rear transverse seam portion (48).

9. Incontinence pad according to one of the preceding claims, **characterized in that** the rear product portion (14) has a maximum transverse extent B2 and the front product portion (16) has a maximum transverse extent B3, wherein a quotient of B2/B3
either a) has a value ≥ 1 and preferably a value ≤ 1.50, preferably ≤ 1.45, preferably ≤ 1.40, preferably ≤ 1.35, preferably ≤ 1.30, preferably ≤ 1.25,
or b) has a value ≤ 1 and preferably ≥ 0.75, preferably ≥ 0.80.

10. Incontinence pad according to one of the preceding claims, **characterized in that** the product length L1 is 450 - 800 mm, preferably 500 - 800 mm, more preferably 500 - 750 mm, and/or **in that** the product width B1 is 200 - 450 mm, preferably 250 - 450 mm, more preferably 280 - 400 mm.

11. Incontinence pad according to one of the preceding claims 1-10, **characterized in that** the rear transverse seam portion (46) has a proportion of 3 - 8%, preferably 4 - 7%, more preferably 4 - 6%, based on a product area of the incontinence pad, and/or **in that** the rear transverse seam portion (46) has a proportion of 45 - 65%, preferably 48 - 63%, more preferably 50 - 60%, based on an area of the rear product end portion (20), in particular **in that** the rear transverse seam portion (46) has an area of 4,000 - 14,000 mm², preferably 5,000 - 12,000 mm², more preferably 5,000 - 11,000 mm².

12. Incontinence pad according to one of the preceding claims 1-11, **characterized in that** the identification means (70) overlaps 15 - 80%, preferably 20 - 70%, more preferably 25 - 60%, more preferably 30 - 50%, of an area of the rear transverse seam portion (46), and/or **in that** the identification means (70) overlaps 8 - 60%, in particular 10 - 55%, more particularly 10 - 50%, more particularly 10 - 40%, of an area of the rear product end portion (20).

13. Incontinence pad according to one of the preceding claims 3-12, **characterized in that** the coloured zone (73) encloses the second identifier (74) starting from a peripheral perimeter (78) of the identification means (70) in such a way that the size of an area A3, which is delimited by the peripheral perimeter (78) of the identification means and an imaginary inner spacing line (110) running within the latter and at a constant first spacing AB1 therefrom, is at least 10%, in particular at least 15%, in particular at least 20%, in particular at least 25%, more particularly at least 30%, of a size of the area A1, more preferably the area A3 is at most 50%, more particularly at most 45%, more particularly at most 40%, more particularly at most 35%, of a size of the area A1, wherein the area A1 is defined as the area inscribed by the peripheral perimeter (78) of the identification means (70), wherein the proportion of the second identifier (74) in the area A3 is at most 10%, in particular at most 7%, more particularly at most 5%, in particular at most 2%, and/or **characterized in that** the size of an area A4, which is delimited by a peripheral perimeter (78) of the identification means (70) and an imaginary spacing line (112) running outside the latter and at a constant second spacing AB2 therefrom, is at least 30%, in particular at least 40%, in particular at least 50%, in particular at least 60%, more particularly is at least 70% of the size of an area A1, wherein the area A1 is defined as the area inscribed by the peripheral perimeter (78) of the identification means (70), wherein preferably at least 90%, in particular at least 93%, in particular at least 95%, of the area A4 is free from further coloured zones, in particular in the form of coatings and/or prints and/or changes in the optical, visual and/or haptic nature particular to the cover sheet material and/or backsheet material.

14. Incontinence pad according to one of the preceding claims 3-13, **characterized in that** the identification means (70) overlaps an area A1 inscribed by a peripheral perimeter (78), and the second identifier (74) takes up a proportion of at most 50%, in particular at most 40%, more particularly at most 30%, more particularly at most 20% and in particular at least 3%, in particular at least 5%, in particular at least 8%, of the area A1 overlapped by the identification means.

15. Incontinence pad according to one of the preceding claims 3-14, **characterized in that** the identification means (70) is inscribed with a peripheral perimeter (78) at its respectively outermost points in a manner tangent to an imaginary rectangle (100), wherein the rectangle (100) is arranged with respect to its edges in the longitudinal and transverse direction of the incontinence article and is divided both in the longitudinal direction and in the transverse direction into in each case 10 same-sized sub-rectangles (101), and, of a number N1 of sub-rectangles (101a) that are located entirely or at least regionally within the peripheral perimeter (78) and a number N2 of those sub-rectangles (101b) that have at least regionally both the first identifier (72) and the second identifier (74), a degree of distribution of N2/N1*100% of at least 10%, in particular at least 20%, in particular at least 25%, in particular at least 30%, in particular at least 35%, more particularly at most 60%, more particularly at most 55%, more particularly at most 50%, more particularly at most 45%, is provided.

## Revendications

1. Protection absorbante jetable pour l'incontinence (2), ayant une direction longitudinale (4) et une direction transversale (6), un axe médian longitudinal (8) et un axe médian transversal (10), et ayant une longueur de produit L1 et une largeur de produit B1, comprenant une portion de produit arrière (14), une portion de produit centrale (15) et une portion de produit avant (16) disposées le long de la direction longitudinale, chaque portion de produit couvrant respectivement un tiers de la longueur de produit L1, comprenant une portion d'extrémité de produit arrière (20) et une portion d'extrémité de produit avant (24), qui couvrent chacune, dans la direction longitudinale, un dixième (1/10) de la longueur de produit L1, comprenant un côté intérieur (28) et un côté extérieur (30), comprenant un matériau de feuille supérieure (32), un matériau de feuille arrière (34) et un corps absorbant (40) disposé entre ceux-ci, un moyen de marquage (70) étant prévu dans la portion d'extrémité de produit arrière (20), lequel est disposé sur l'axe médian longitudinal (8), une section de couture transversale arrière (46) étant prévue à l'intérieur de la portion d'extrémité de produit arrière (20), la section de couture transversale arrière (46) comprenant un débord (38) ou étant formée de celui-ci, le débord (38) comprenant une zone (39) ou étant formé de celle-ci, dans laquelle le matériau de feuille supérieure (32) et/ou le matériau de feuille arrière (34) s'étend à l'extérieur d'une ligne de contour (42) délimitant le corps absorbant (40), le moyen de marquage (70) étant disposé, au moins par zones, dans la section de couture transversale arrière (46), à l'intérieur de la portion d'extrémité de produit arrière (20), dans lequel le moyen de marquage (70) présente, dans une première zone (21) de la portion d'extrémité de produit arrière (20), un premier marquage (72), qui est formé par une zone colorée s'étendant sur une surface (73) et qui fournit une première information, la zone colorée (73) étant visuellement distincte d'une deuxième zone (22) de la portion d'extrémité de produit arrière (20) entourant le premier marquage (72), et le moyen de marquage (70) étant visuellement perceptible, au moins par zones, à la fois sur le côté intérieur (28) et sur le côté extérieur (30),
**caractérisée en ce que**, sur le côté intérieur (28) ou sur le côté extérieur (30), le moyen de marquage (70) présente une surface A1 ayant une extension transversale maximale E1,
- et **en ce que** le moyen de marquage est exactement inscrit dans un premier rectangle imaginaire (200), le premier rectangle imaginaire (200) présentant des premier et deuxième côtés longitudinaux (202, 203) parallèles à la direction longitudinale (4), ainsi qu'un premier côté transversal intérieur (204) et un côté transversal extérieur (205) parallèles à la direction transversale (6), le premier côté transversal intérieur (204) étant situé à une première distance D1 d'une extrémité arrière du produit (11), et les premier et deuxième côtés longitudinaux (204, 205) ayant une longueur Z1,
- et **en ce qu'**un deuxième rectangle imaginaire (210) peut être positionné sur le même côté intérieur (28) ou le même côté extérieur (30) dans la portion d'extrémité de produit avant (24), le deuxième rectangle imaginaire (210) présentant un troisième et un quatrième côtés longitudinaux (212, 213) parallèles à la direction longitudinale (4), ainsi qu'un deuxième côté transversal intérieur (214) et un deuxième côté transversal extérieur (215) parallèles à la direction transversale (6), les troisième et quatrième côtés longitudinaux (212, 213) ayant une longueur Z1' correspondant à la longueur Z1 (Z1' = Z1), le deuxième côté transversal intérieur (214) étant situé à une distance D2 d'une extrémité avant du produit (12), et **en ce que**, pour chacune des positions dans lesquelles le deuxième rectangle imaginaire (210) est positionné, il est vérifié que :
a) soit aucune marque colorée (220) visuellement perceptible n'est présente à l'intérieur du deuxième rectangle imaginaire (210), soit
b) dans le cas où une marque colorée (220) visuellement perceptible est présente à l'intérieur du deuxième rectangle imaginaire (210), la marque colorée (220) présente une surface A2 représentant une proportion surfacique d'au plus 25 %, notamment d'au plus 20 %, plus particulièrement d'au plus 15 %, encore plus particulièrement d'au plus 10 % de la surface A1. et/ou
c) dans le cas où une marque colorée (220) visuellement perceptible est présente à l'intérieur du deuxième rectangle imaginaire (210), la marque colorée (220) présente une extension transversale maximale E2, l'extension transversale maximale E2 étant inférieure à l'extension transversale maximale E1 du moyen de marquage (70), l'extension transversale maximale E2 représentant notamment au plus 40 %, en particulier au plus 30 %, plus particulièrement au plus 20 %, encore plus particulièrement au plus 10 % de l'extension transversale maximale E1,
- les positions dans lesquelles le deuxième rectangle imaginaire (210) est positionné étant limitées **en ce que** le deuxième rectangle imaginaire (210), par rapport au premier rectangle imaginaire (200), est déplacé parallèlement à l'axe médian longitudinal (8), et **en ce que** la relation suivante s'applique à la distance D2 :
a) D2 ≤ 1,5 x D1 et
b) le deuxième côté transversal extérieur (215) du deuxième rectangle imaginaire (210) ne s'étend pas au-delà de l'extrémité avant du produit (12).

2. Protection absorbante pour l'incontinence selon l'une quelconque des revendications précédentes, **caractérisée en ce que** le moyen de marquage (70) est un élément unique.

3. Protection absorbante pour l'incontinence selon l'une quelconque des revendications précédentes, **caractérisée en ce que** le moyen de marquage (70) comprend un deuxième marquage (74), qui fournit une deuxième information, lequel est formé par la présence d'évidements (76) de la zone colorée (73) dans le premier marquage (72), le deuxième marquage (74) comprenant notamment des caractères alphanumériques.

4. Protection absorbante pour l'incontinence selon l'une quelconque des revendications précédentes, **caractérisée en ce que** le moyen de marquage (70) est appliqué sur le matériau de feuille arrière (34), sur une face supérieure du matériau de feuille arrière (34) tournée vers le côté intérieur (28) ou sur une face supérieure tournée vers le côté extérieur (30), ou, notamment dans le cas d'un matériau de feuille arrière (34) comprenant une couche interne de film (35) et une couche externe de non-tissé (36), le moyen de marquage (70) est disposé entre celles-ci et, notamment, sur la face supérieure de la couche de film (35) tournée vers le côté extérieur (30), ou **en ce que** le moyen de marquage (70) est appliqué sur le matériau de feuille supérieure (32), notamment sur une face supérieure du matériau de feuille supérieure (32) tournée vers le côté extérieur (30).

5. Protection absorbante pour l'incontinence selon l'une quelconque des revendications précédentes, **caractérisée en ce que** la zone colorée (73) du moyen de marquage (70) est une impression, notamment une impression en couleur.

6. Protection absorbante pour l'incontinence selon l'une quelconque des revendications précédentes, **caractérisée en ce que** le moyen de marquage (70), respectivement la zone colorée (73) du premier marquage (74) du moyen de marquage, présente une extension transversale maximale E1 d'au plus 1/2, notamment d'au plus 1/3, notamment d'au plus ¼ d'une extension transversale maximale B2 de la portion de produit arrière (14), et notamment d'au moins 1/6 d'une extension transversale maximale B2 de la portion de produit arrière (14).

7. Protection absorbante pour l'incontinence selon l'une quelconque des revendications précédentes, **caractérisée en ce qu'**une extension transversale maximale E1 du moyen de marquage (70) dans la direction transversale (6) de la protection absorbante pour l'incontinence est supérieure à une extension longitudinale maximale F1 du moyen de marquage dans la direction longitudinale (4) de la protection absorbante pour l'incontinence.

8. Protection absorbante pour l'incontinence selon l'une quelconque des revendications précédentes, **caractérisée en ce que** le moyen de marquage (70) présente une extension longitudinale maximale F1 et est disposé, sur au moins 50 % de l'extension longitudinale maximale F1, de préférence au moins 60 %, plus préférablement au moins 70 %, encore plus préférablement au moins 80 %, encore plus préférablement au moins 90 %, à l'intérieur d'une section de couture transversale arrière (46), le moyen de marquage (70) étant de préférence entièrement disposé à l'intérieur d'une section de couture transversale arrière (48).

9. Protection absorbante pour l'incontinence selon l'une quelconque des revendications précédentes, **caractérisée en ce que** la portion de produit arrière (14) présente une extension transversale maximale B2 et la portion de produit avant (16) présente une extension transversale maximale B3, un quotient de B2/B3
soit a) présentant une valeur ≥ 1 et de préférence une valeur ≤ 1,50, de préférence ≤ 1,45, de préférence ≤ 1,40, de préférence ≤ 1,35, de préférence ≤ 1,30, de préférence ≤ 1,25,
soit b) présentant une valeur ≤ 1 et de préférence ≥ 0,75, de préférence ≥ 0,80.

10. Protection absorbante pour l'incontinence selon l'une quelconque des revendications précédentes, **caractérisée en ce que** la longueur de produit L1 est de 450 à 800 mm, de préférence de 500 à 800 mm, plus préférablement de 500 à 750 mm, et/ou **en ce que** la largeur de produit B1 est de 200 à 450 mm, de préférence de 250 à 450 mm, plus préférablement de 280 à 400 mm.

11. Protection absorbante pour l'incontinence selon l'une quelconque des revendications précédentes 1 à 10, **caractérisée en ce que** la section de couture transversale arrière (46) présente une proportion surfacique de 3 à 8 %, de préférence de 4 à 7 %, plus préférablement de 4 à 6 %, par rapport à une surface du produit de la protection absorbante pour l'incontinence, et/ou **en ce que** la section de couture transversale arrière (46) présente une proportion surfacique de 45 à 65 %, de préférence de 48 à 63 %, plus préférablement de 50 à 60 %, par rapport à une surface de la portion d'extrémité de produit arrière (20), notamment **en ce que** la section de couture transversale arrière (46) présente une surface de 4 000 à 14 000 mm², de préférence de 5 000 à 12 000 mm², plus préférablement de 5 000 à 11 000 mm².

12. Protection absorbante pour l'incontinence selon l'une quelconque des revendications précédentes 1 à 11, **caractérisée en ce que** le moyen de marquage (70) recouvre 15 à 80 %, de préférence 20 à 70 %, plus préférablement 25 à 60 %, encore plus préférablement 30 à 50 % d'une surface de la section de couture transversale arrière (46), et/ou **en ce que** le moyen de marquage (70) recouvre 8 à 60 %, notamment 10 à 55 %, plus particulièrement 10 à 50 %, encore plus particulièrement 10 à 40 % d'une surface de la portion d'extrémité de produit arrière (20).

13. Protection absorbante pour l'incontinence selon l'une quelconque des revendications précédentes 3 à 12, **caractérisée en ce que** la zone colorée (73), à partir d'une ligne de contour périphérique (78) du moyen de marquage (70), entoure le deuxième marquage (74) de telle manière que la taille d'une surface A3, qui est délimitée par la ligne de contour périphérique (78) du moyen de marquage et par une ligne de distance intérieure imaginaire (110) s'étendant à l'intérieur de celle-ci et à une première distance constante AB1 de celle-ci, représente au moins 10 %, notamment au moins 15 %, notamment au moins 20 %, notamment au moins 25 %, plus particulièrement au moins 30 % de la taille de la surface A1, la surface A3 représentant de préférence au plus 50 %, plus particulièrement au plus 45 %, plus particulièrement au plus 40 %, plus particulièrement au plus 35 % de la taille de la surface A1, la surface A1 étant définie comme la surface inscrite par la ligne de contour périphérique (78) du moyen de marquage (70), la proportion surfacique du deuxième marquage (74) par rapport à la surface A3 étant d'au plus 10 %, notamment d'au plus 7 %, plus particulièrement d'au plus 5 %, notamment d'au plus 2 %, et/ou **caractérisée en ce que** la taille d'une surface A4, qui est délimitée par une ligne de contour périphérique (78) du moyen de marquage (70) et par une ligne de distance imaginaire (112) s'étendant à l'extérieur de celle-ci et à une deuxième distance constante AB2 de celle-ci, représente au moins 30 %, notamment au moins 40 %, notamment au moins 50 %, notamment au moins 60 %, plus particulièrement au moins 70 % de la taille d'une surface A1, la surface A1 étant définie comme la surface inscrite par la ligne de contour périphérique (78) du moyen de marquage (70), au moins 90 %, notamment au moins 93 %, notamment au moins 95 % de la surface A4 étant de préférence exempts d'autres zones colorées, notamment de revêtements et/ou d'impressions et/ou de modifications de la nature optique visuelle et/ou haptique propre au matériau de feuille supérieure et/ou au matériau de feuille arrière.

14. Protection absorbante pour l'incontinence selon l'une quelconque des revendications précédentes 3 à 13, **caractérisée en ce que** le moyen de marquage (70) recouvre une surface A1 inscrite par une ligne de contour périphérique (78), et **en ce que** le deuxième marquage (74) occupe une proportion surfacique d'au plus 50 %, notamment d'au plus 40 %, plus particulièrement d'au plus 30 %, plus particulièrement d'au plus 20 %, et notamment d'au moins 3 %, notamment d'au moins 5 %, notamment d'au moins 8 % de la surface A1 recouverte par le moyen de marquage.

15. Protection absorbante pour l'incontinence selon l'une quelconque des revendications précédentes 3 à 14, **caractérisée en ce que** le moyen de marquage (70) est inscrit de manière tangente, par une ligne de contour périphérique (78), dans un rectangle imaginaire (100) au niveau de ses points les plus extérieurs respectifs, le rectangle (100) étant orienté, par ses côtés, dans les directions longitudinale et transversale de l'article pour l'incontinence et étant subdivisé, tant dans la direction longitudinale que dans la direction transversale, en respectivement 10 sous-rectangles (101) de taille identique, un degré de répartition de N2/N1*100 % d'au moins 10 %, notamment d'au moins 20 %, notamment d'au moins 25 %, notamment d'au moins 30 %, notamment d'au moins 35 %, et plus particulièrement d'au plus 60 %, plus particulièrement d'au plus 55 %, plus particulièrement d'au plus 50 %, plus particulièrement d'au plus 45 %, étant prévu à partir d'un nombre N1 de sous-rectangles (101a) qui sont localisés entièrement ou au moins partiellement à l'intérieur de la ligne de contour périphérique (78), et d'un nombre N2 de ceux des sous-rectangles (101b) qui présentent au moins partiellement à la fois le premier marquage (72) et le deuxième marquage (74).
